# EUROPEAN PATENT SPECIFICATION

(11) **EP 1 861 114 B1**
(45) Date of publication and mention of the grant of the patent: **02.02.2011**
(21) Application number: 06738066.7
(22) Date of filing: 13.03.2006
(51) Int. Cl.: A61P 27/02, A61K 38/00

(54) **COMPOSITIONS COMPRISING MODULATORS OF G-PROTEIN-COUPLED RECEPTOR FOR TREATMENT OF MACULAR DEGENERATION**
ZUSAMMENSETZUNGEN MIT MODULATOREN DES G-PROTEIN-GEKOPPELTEN REZEPTORS ZUR BEHANDLUNG VON MAKULADEGENERATION
COMPOSITIONS RENFERMANT DES MODULATEURS DU RECEPTEUR LIÉ A LA PROTÉINE G ET DESTINEES AU TRAITEMENT DE LA DEGENERESCENCE MACULAIRE

(30) Priority: 11.03.2005 US 660752 P
(43) Date of publication of application: 05.12.2007
(73) Proprietor: Potentia Pharmaceuticals, Inc., Louisville, KY 40202 (US)
(72) Inventor: DESCHATELETS, Pascal, Louisville, Kentucky 40241 (US); FRANCOIS, Cedric, Louisville, Kentucky 40204 (US)
(74) Representative: Crooks, Elizabeth Caroline
(86) International application number: PCT/US2006/008960
(87) International publication number: WO 2006/099330

(56) References cited:
- WO-A-03/086448
- WO-A2-01/84149
- WO-A2-2004/041160
- US-B1- 6 821 950
- FUKUOKA Y; STRAINIC M; MEDOF M E: "Differential cytokine expression of human retinal pigment epithelial cells in response to stimulation by C5a" CLINICAL AND EXPERIMENTAL IMMUNOLOGY, vol. 131, no. 2, February 2003 (2003-02), pages 248-253, XP002417172 ISSN: 0009-9104
- ROTH FELIX; BINDEWALD ALMUT; HOLZ FRANK G: "Keypathophysiologic pathways in age-related macular disease" GRAEFE'S ARCHIVE FOR CLINICAL AND EXPERIMENTAL OPHTHALMOLOGY, vol. 242, no. 8, August 2004 (2004-08), pages 710-716, XP002417173 ISSN: 0721-832X

## Description

### Cross-Reference to Related Application

This application claims priority to and the benefit of U.S..Provisional Patent Application Serial Number 60/660,752, filed March 11, 2005, which is incorporated herein by reference.

### Background of the Invention

The macula is a small area in the retina, approximately 3 to 5 millimeters in size, adjacent to the optic nerve. It is the most sensitive area of the retina and contains the fovea, a depressed region that allows for high visual acuity and contains a dense concentration of cones, the photoreceptors that that are responsible for color vision. Macular degeneration is a term that refers to a number of different diseases characterized by degenerative changes in the macula, all of which lead to a loss of central vision.

Age-related macular degeneration (ARMD or AMD) is the most common cause of functional blindness in developed countries for those over 50 years of age (Seddon, JM. Epidemiology of age-related macular degeneration. In: Ogden, TE, et al., eds. Ryan SJ, ed-in-chief. Retina Vol II. 3rd ed. St. Louis, MO: Mosby; 2001:1039-50). The disease is characterized by progressive degeneration of the retina, retinal pigment epithelium (RPE), and underlying choroid (the highly vascular tissue that lies beneath the RPE, between the retina and the sclera). The retinal pigment epithelial layer is believed to be crucial for photoreceptor health. Cells in this layer recycle visual pigment (rhodopsin), phagocytose photoreceptor tips daily as part of rod and cone regeneration, and transport fluid across the membrane to the choroid, which is believed to help prevent detachment of the neural retina. Central vision deteriorates when cells in the RPE cease to function properly, which can lead to photoreceptor degeneration.

ARMD has been classified into both "dry" and "wet" (exudative) forms. Dry ARMD is much more common than wet ARMD, but the dry form can progress to the wet form, and the two occur simultaneously in a significant number of cases. Dry ARMD is characterized by progressive apoptosis of cells in the RPE layer, overlying photoreceptor cells, and frequently also the underlying cells in the choroidal capillary layer. Confluent areas (typically at least 175 µm in minimum diameter) of RPE cell death accompanied by overlying photoreceptor atrophy are referred to as geographic atrophy. Patients with this form of ARMD experience a slow and progressive deterioration in central vision. Wet ARMD can be responsible for sudden and disabling loss of vision due, for example, to bleeding or fluid from abnormal vessels that have grown from the choriocapillaris beneath the RPE and the macula. A subtype of neovascular ARMD in which angiomatous proliferation originates from the retina and extends posteriorly into the subretinal space, eventually communicating in some cases with choroidal new vessels has been identified (Yannuzzi, L.A., et al., Retina, 21(5);416-34, 2001). This form of neovascular ARMD, termed retinal angiomatous proliferation (RAP) can be particularly severe. The existence of macular drusen is a strong risk factor for the development of both wet and dry forms of ARMD (Ambati, J., et al., *supra*).

Despite extensive investigation, the pathogenesis of ARMD remains unclear, and the etiology of the molecular events that occur is not well understood. Treatment options are limited, and none are fully effective. The standard of care in exudative ARMD is laser photocoagulation. However, while laser photocoagulation of subfoveal neovascularization can limit visual loss, it also results in an absolute central scotoma and eliminates any possibility of central vision recovery. Photodynamic therapy is a more recent approach, but its efficacy is limited. While surgical approaches including submacular surgery and macular translocation show promise, these are still in a developmental stage and carry attendant risks. Even partially effective treatments for the dry form of ARMD are essentially lacking. Thus there is a need in the art for new approaches to the treatment of ARMD. In addition, there is a need in the art for new approaches to the treatment of other diseases and conditions characterized by macular degeneration and/or choroidal neovascularation.

### Summary of the Invention

The present invention addresses the foregoing needs, among others. The invention provides a composition for use in treating a subject at risk of or suffering from a macular degeneration related condition or ocular neovascularization comprising a compound that is an antagonist of a G protein coupled receptor (GPCR). In particular, the invention provides a composition for use in treating a subject suffering from or at risk of a macular degeneration related condition or ocular neovascularization comprising a compound having general formula I below:

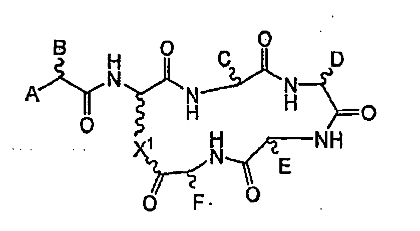

where A is H, alkyl, aryl, NH₂, NHalkyl, N(alkyl)₂, NHaryl or NHacyl; B is an alkyl, aryl, phenyl, benzyl, naphthyl or indole group, or the side chain of a D- or L-amino acid selected from the group consisting of phenylalanine, homophenylalanine, tryptophan, homotryptophan, tyrosine, and homotyrosine; C is * the side chain of a D-, L- or homo-amino acid selected from the group consisting of proline, alanine, leucine, valine, isoleucine, arginine, histidine, aspartate, glutamate, glutamine, asparagine, lysine, tyrosine, phenylalanine, cyclohexylalanine, norleucine, tryptophan, cysteine and methionine; D is the side chain of a D- or L-amino acid selected from the group consisting of cyclohexylalanine, homocyclohexylalanine, leucine, norleucine, homoleucine, homonorleucine and tryptophan; E is the side chain of a D- or L-amino acid selected from the group consisting of tryptophan and homotryptophan; F is the side chain of a D- or L-amino acid selected from the group consisting of arginine, homoarginine, lysine and homolysine or is one of the following side-chains

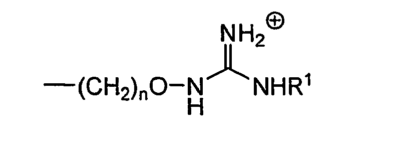

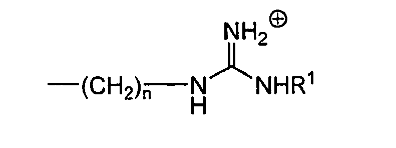

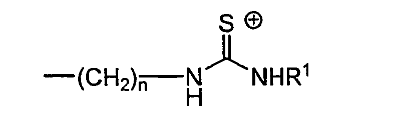

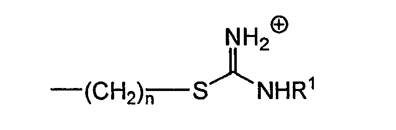

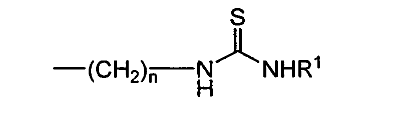

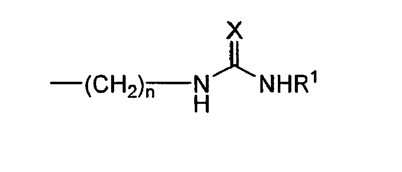

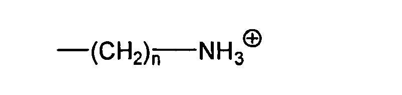

or another mimetic of an arginine side chain, where X is NCN, NNO₂, CHNO₂ or NSO₂NH₂; n is an integer from 1 to 4, and R¹ is H or an alkyl, aryl, CN, NH₂, OH, --CO--CH₂CH₃, --CO-CH₃, --CO--CH₂CH₂CH₃, --CO--CH₂ Ph, or --CO-Ph; and X¹ is --(CH₂)ₙNH-- or (CH₂)ₙ -S--, --(CH₂)₂ O--, --(CH₂)₃ O-, --(CH₂)₃ --,-(CH₂)₄ --, or --CH₂ COCHRNH--, where R is the side chain of any common or uncommon amino acid, and
where n is an integer of from 1 to 4, e.g., 1, 2, 3, or 4.

In certain embodiments of the invention F is one of the following side-chains: 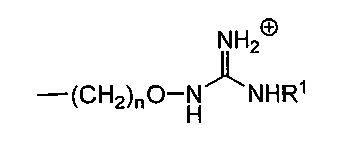 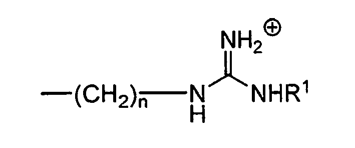 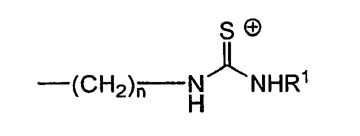 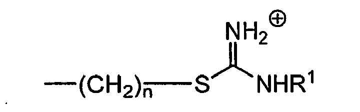 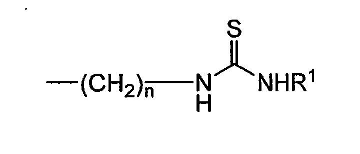 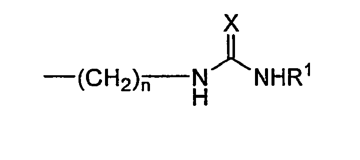 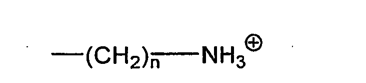 or another mimetic of an arginine side chain; where X is NCN, NNO₂, CHNO₂ or NSO₂NH₂; n is an integer from 1 to 4, and R¹ is H or an alkyl, aryl, CN, NH₂, OH,CO--CH₂CH₃, -CO--CH₃, -CO-CH₂CH₂CH₃, --CO--CH₂ Ph, or -CO-Ph; B is an indole, indole methyl, benzyl, phenyl, naphthyl, naphthyl methyl, cinnamyl group, or any other derivative of the aromatic group; and C is D- or L-cyclohexylalanine (Cha), leucine, valine, isoleucine, phenylalanine, tryptophan or methionine. In certain embodiments of the invention A is L-arginine. In certain embodiments of the invention F is an L-amino acid. In certain embodiments F is L-arginine. In certain embodiments n =1, 2, 3, or 4.

In certain embodiments of the invention the compound is selected from the group consisting of SEQ ID NOs: 11, 12, 13, 14,15,16,17, 18,19, 20, 21, 22, 23, 24, 25, 26, 27 and 28, as described herein and in U.S. Pat. No. 6,821,950. Other embodiments disclosed therein may also be used. For example, A, B, C, D, E, F, and R¹ may be any of the groups mentioned in U.S. Pat. No. 6,821,950. It is noted that the letters A, B, C, D, E, and F in the formulas presented herein are to be given the meanings described herein and in U.S. Pat. No. 6,821,950 and do not stand for chemical elements or isotopes such as boron, carbon, deuterium, or fluorine. The compounds described herein will be referred to collectively herein as GPCRAs, i.e., an individual compound described herein is referred to as a GPCRA.

The invention further provides a GPCRA for use in preventing or treating an ocular inflammatory condition in a subject suffering from or at risk of an ocular inflammatory condition.

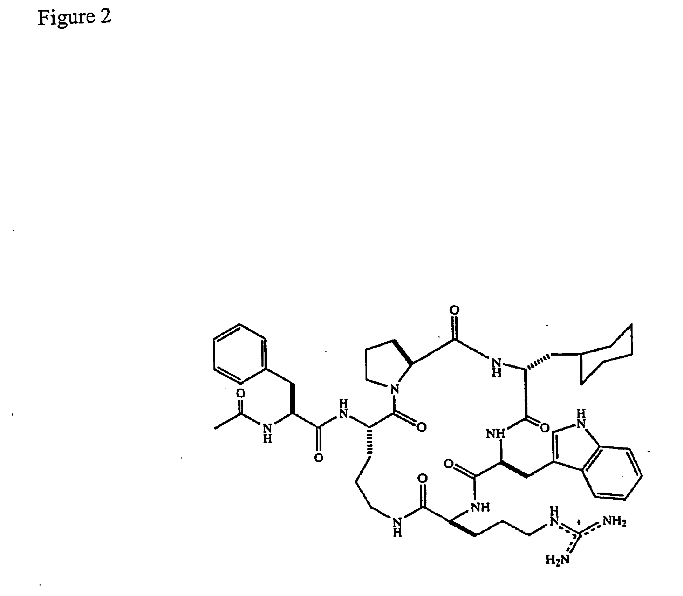

The invention further provides a composition comprising a compound having the formula shown in Figure 2 for use in preventing or treating a macular degeneration related condition or ocular neovascularization in a subject suffering from or at risk of a macular degeneration related condition or ocular neovascularization.

The invention further provides a compound having the formula shown in Figure 2 for use in preventing or treating an ocular inflammatory condition in a subject suffering from or at risk of an ocular inflammatory condition.

The GPCRA may be an antagonist of the C5a receptor.

The subject may be in need of treatment for a macular degeneration related condition or ocular neovascularization.

The subject may be in need of treatment for an ocular inflammatory condition.

Methods for making the GPCRAs, confirming their structure, and testing their activity as modulators of a GPCR are disclosed in U.S. Pat No. 6,281,950, March, DR, et al., Mol. Pharmacol., 65(4),2004, and in Woodruff, TM, et al., J Pharmaco/ Exp Ther., 314(2):811-7, 2005. The present invention encompasses use of any such compounds that act as antagonists of the C5a receptor for treatment of a macular degeneration related condition,ocular neovascularization, or an ocular inflammatory condition. In general, these compounds may be administered to a subject in a similar fashion to the viral complement control proteins described in provisional patent application Ser. No. 60/616,983, entitled "Treatment of Macular Degeneration and Related Conditions with Poxvirus Complement Control Proteins" and U.S.S.N.11/247,886, filed Oct. 8, 2005, entitled "VIRAL COMPLEMENT CONTROL PROTEINS FOR EYE DISORDERS". For example, the invention provides compositions (e.g., sustained release formulations), ocular implants, and polymeric delivery vehicles comprising one or more of the GPCRA. The compositions and polymeric delivery vehicles may further comprise a moiety that binds to a component present in the eye of a subject at risk of or suffering from a macular degeneration related condition, ocular neovascularization, and/or ocular inflammation. The compound or polymeric delivery vehicle may be covalently or noncovalently attached to the binding moiety.

In certain embodiments of the invention the composition is administered locally, e.g., by injection into the eye. In certain embodiments the composition is administered locallyy by injection or surgical implantation of an ocular implant or polymeric delivery vehicle (e.g., nanoparticles or microparticles) from which the compound is released. Exemplary methods include intravitreal administration, subtenon or retrobulbar administration, and juxtascleral administration. Any method of introducing the composition, ocular implant, or polymeric delivery vehicle into the eye may be used to administered the compound. In other embodiments of the invention the composition is administered systemically (e.g., intravenously, orally, etc.).

Methods for testing the inventive compositions, implants, and polymeric delivery vehicles are also provided.

Unless otherwise stated, the invention makes use of standard methods of organic chemistry, molecular biology, cell culture, animal maintenance, ophthalmologic examination, and administration of therapeutic agents to subjects, etc., and uses art-accepted meanings of terms. This application refers to various patents and publications. The contents of all of these are incorporated by reference. In addition, the following publications are incorporated herein by reference: Current Protocols in Molecular Biology, Current Protocols in Immunology, Current Protocols in Protein Science*,* and Current Protocols in Cell Biology, all John Wiley & Sons, N.Y., edition as of July 2002; Sambrook, Russell, and Sambrook, Molecular Cloning A Laboratory Manual, 3rd ed., Cold Spring Harbor Laboratory Press, Cold Spring Harbor, 2001; Kuby Immunology, 4th ed., Goldsby, R.A., Kindt, T.J., and Osborne, B. (eds.), W.H. Freeman, 2000, Goodman and Gilman's The Pharmacological Basis of Therapeutics, 10th Ed..McGraw Hill, 2001, and Katzung, B. (ed.) Basic and Clinical Pharmacology, McGraw-Hill/Appleton & Lange; 8th edition (September 21, 2000). In the event of a conflict between any of the incorporated references and the instant specification, the specification shall control. The determination of whether a conflict or inconsistency exists is within the discretion of the inventors and can be made at any time.

### Brief Description of the Drawing

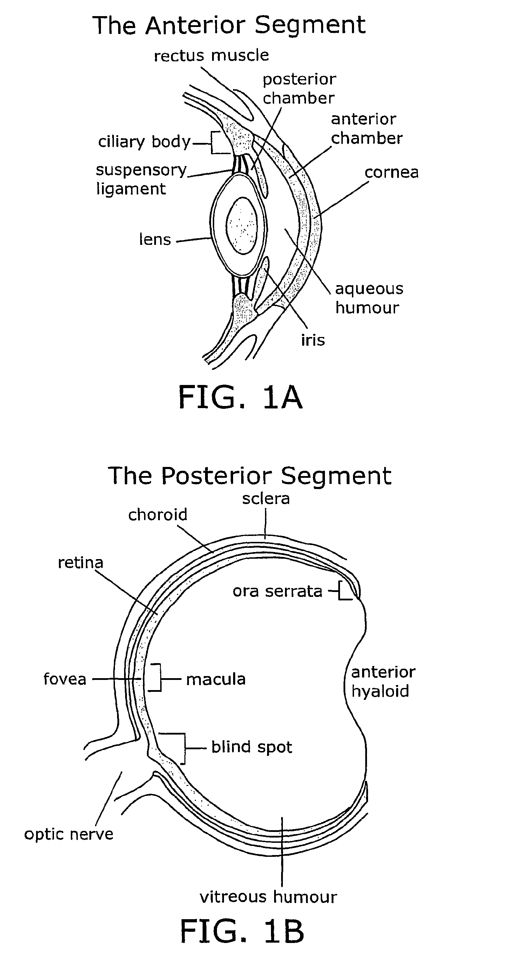

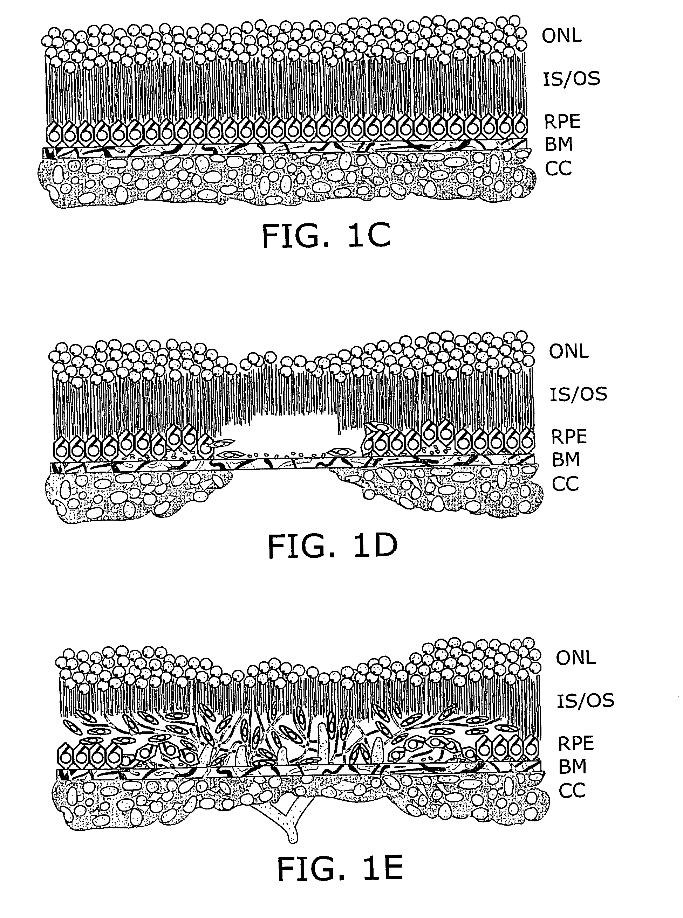

Figures 1A-1E show schematic representations of the anterior and posterior segments of the eye (1A and 1B) and the outer layers of the eye (1C-1E). Figure 1C depicts a normal eye. Figure 1D depicts an eye suffering from dry ARMD. Figure 1E depicts an eye suffering from exudative ARMD. ONL = outer nuclear layer; IS = inner segment; OS = outer segment; RPE = retinal pigment epithelial layer; BM = Bruch's membrane; CC = choriocapillaris. From Tezel, T., et al., Trends in Molecular Medicine, 10(9), 417-420, 2004.

Figure 2 shows an exemplary compound for use in methods and compositions of the invention.

### Definitions

"Approximately" or "about" in reference to a number generally includes numbers that fall within a range of 10% of the number in either direction (greater than or less than) unless otherwise stated or otherwise evident from the context (except where such number would exceed 100% of a possible value). Where ranges are stated, the endpoints are included within the range unless otherwise stated or otherwise evident from the context.

"Biocompatible" refers to a material that is substantially non-toxic to cells *in vitro,* e.g., if its addition to cells in culture results in less than or equal to 20% cell death. A material is considered biocompatible with respect to a recipient if it is substantially nontoxic to the recipient's cells in the quantities and at the location used, and also does not elicit or cause a significant deleterious or untoward effect on the recipient's body, e.g., an immunological or inflammatory reaction, unacceptable scar tissue formation, etc.

"Biodegradable" means that a material is capable of being broken down physically and/or chemically within cells or within the body of a subject, e.g., by hydrolysis under physiological conditions, by natural biological processes such as the action of enzymes present within cells or within the body, etc., to form smaller chemical species which can be metabolized and, optionally, reused, and/or excreted or otherwise disposed of. Preferably a biodegradable compound is biocompatible.

"Choroidal neovascularization" (CNV) refers to the abnormal development of blood vessels arising from the choriocapillaris. The blood vessels typically extend through Bruch's membrane, RPE layer, and/or subretinal space.

A "complement component" or "complement protein" is a molecule that is involved in activation of the complement system or participates in one or more complement-mediated activities. Components of the classical complement pathway include, e.g., C1q, C1r, C1s, C2, C3, C4, C5, C6, C7, C8, C9, and the C5b-9 complex, also referred to as the membrane attack complex (MAC) and active fragments or enzymatic cleavage products of any of the foregoing (e.g., C3a, C3b, C4a, C4b, C5a, etc.). Components of the alternative pathway include, e.g., factors B, D, H, and I, and properdin. Components of the lectin complement pathway include mannose-binding lectin (MBL) and MBL-associated serine protease (MASP). Complement components include receptors for any of the afore-mentioned components of the classical, alternative, or lectin complement pathways.

"Concurrent administration" as used herein with respect to two or more agents, e.g., therapeutic agents, is administration performed using doses and time intervals such that the administered agents are present together within the body, or at a site of action in the body such as within the eye) over a time interval in less than *de minimis* quantities, i.e., in quantities sufficient to have a detectable biological effect or response. The time interval can be minutes, hours, days, weeks, etc. Accordingly, the agents may, but need not be, administered together as part of a single composition. In addition, the agents may, but need not be, administered simultaneously (e.g., within less than 5 minutes, or within less than 1 minute) or within a short time of one another (e.g., less than 1 hour, less than 30 minutes, less than 10 minutes, approximately 5 minutes apart). According to various embodiments of the invention agents administered within such time intervals may be considered to be administered at substantially the same time. One of ordinary skill in the art will be able to readily determine appropriate doses and time interval between administration of the agents so that they will each be present at more than *de minimis* levels within the body or, preferably, at effective concentrations within the body. When administered concurrently, the effective concentration of each of the agents to elicit a particular biological response may be less than the effective concentration of each agent when administered alone, thereby allowing a reduction in the dose of one or more of the agents relative to the dose that would be needed if the agent was administered as a single agent. The effects of multiple agents may, but need not be, additive or synergistic. The agents may be administered multiple times.

An "effective amount" of an active agent refers to the amount of the active agent sufficient to elicit a desired biological response. As will be appreciated by those of ordinary skill in this art, the absolute amount of a particular agent that is effective may vary depending on such factors as the desired biological endpoint, the agent to be delivered, the target tissue, *etc*. Those of ordinary skill in the art will further understand that an "effective amount" may be administered in a single dose, or may be achieved by administration of multiple doses. For example, an effective amount may be an amount sufficient to achieve one or more of the following; (i) prevent drusen formation; (ii) cause a reduction in drusen number and/or size (drusen regression); (iii) cause a reduction in or prevent lipofuscin deposits; (iv) prevent visual loss or slow the rate of visual loss; (v) prevent or slow the rate of choroidal neovascularization; (vi) cause a reduction in size and/or number of lesions characterized by choroidal neovascularization; (vii) improve visual acuity and/or contrast sensitivity; (viii) prevent or reduce the rate of photoreceptor or RPE cell atrophy or apoptosis; (ix) prevent or slow progression from the dry to the wet form of ARMD.

The term "isolated" means 1) separated from at least some of the components with which it is usually associated in nature; 2) prepared or purified by a process that involves the hand of man; and/or 3) not occurring in nature.

The term "linked", when used with respect to two or more moieties, means that the moieties are physically associated or connected with one another to form a structure that is sufficiently stable so that the moieties remain associated under the conditions in which the structure is formed and, preferably, under the conditions in which it is used, e.g., physiological conditions. In certain preferred embodiments of the invention the association is a covalent linkage (where "linkage" refers to a connection between two moieties). In other embodiments the association is noncovalent. Moieties may be linked either directly or indirectly. When two moieties are directly linked, they are either covalently bonded to one another or are in sufficiently close proximity such that intermolecular forces between the two moieties maintain their association. Formation of the covalent bond may result in removal of one or more atoms from either or both of the moieties (e.g., loss of an H, OH, etc.) but does not result in incorporation of additional atoms not present in either of the moieties. When two moieties are indirectly linked, they are each linked either covalently or noncovalently to a third moiety, which maintains the association between the two moieties. In general, when two moieties are referred to as being linked by a "linker" or "linking moiety" or "linking portion", the association between the two linked moieties is indirect, and typically each of the linked moieties is covalently bonded to the linker. The linker can be any suitable moiety that reacts with the two moieties to be linked within a reasonable period of time, under conditions consistent with stability of the moieties (which may be protected as appropriate, depending upon the conditions), and in sufficient amount, to produce a reasonable yield. It will be appreciated that certain linkages are selected to be unstable under particular conditions. For example, they may be subject to acid or base-catalyzed hydrolysis and/or subject to cleavage by a specific enzyme or other reagent. Such linkages are referred to herein as "cleavable". A linkage is considered cleavable for purposes of the present invention if it is cleaved in a structure-specific manner by conditions likely to be present intra or extracellularly somewhere inside the body, such as appropriate temperature, pH, and/or the presence of particular ions or molecules (e.g., hydrolytic enzymes).

"Liposomes" are artificial microscopic spherical particles in an aqueous medium, formed by a lipid bilayer (or multilayers) enclosing an aqueous compartment. Liposomes are commonly used in molecular biology as a delivery vehicle for various types of molecules (such as proteins, small molecules, DNA, and RNA), including a number of different drugs and can be used for delivering the compositions of the invention.

"Local administration" or "local delivery", in reference to delivery of a composition, formulation, or device of the invention, refers to delivery that does not rely upon transport of the agent to its intended target tissue via the vascular or lymphatic system from a site of adminstration that is remote from the intended target tissue. The agent is delivered directly to its intended target tissue or in the vicinity thereof, e.g. by injection or implantation. It will be appreciated that a small amount of the delivered agent may enter the vascular system and may ultimately reach the target tissue via the vascular system.

"Macular degeneration related condition" refers to any of a number of disorders and conditions in which the macula degenerates or loses functional activity. The degeneration or loss of functional activity can arise as a result of, for example, cell death, decreased cell proliferation, loss of normal biological function, or a combination of the foregoing. Macular degeneration can lead to and/or manifest as alterations in the structural integrity of the cells and/or extracellular matrix of the macula, alteration in normal cellular and/or extracellular matrix architecture, and/or the loss of function of macular cells. The cells can be any cell type normally present in or near the macula including RPE cells, photoreceptors, and capillary endothelial cells. ARMD is the major macular degeneration related condition, but a number of others are known including, but not limited to, Best macular dystrophy, Sorsby fundus dystrophy, Mallatia Leventinese and Doyne honeycomb retinal dystrophy.

"Marker", for the purpose of the description of the invention, may refer to any molecular moiety (e.g., protein, peptide, mRNA or other RNA species, DNA, lipid, carbohydrate) that characterizes, indicates, or identifies a particular diseased or physiological state (e.g., apoptotic, cancerous, normal) or characterizes, indicates, or identifies one or more cell type(s), tissue type(s), or embryological origin. The presence or absence of certain marker(s), or the amount of certain marker(s), may indicate a particular physiological or diseased state of a patient, organ, tissue, or cell. A cellular marker may, but need not be, cell type specific. For example, a cell type specific marker is generally a protein, peptide, mRNA, lipid, or carbohydrate that is present at a higher level on or in a particular cell type or cell types of interest than on or in many other cell types. In some instances a cell type specific marker is present at detectable levels only on or in a particular cell type of interest. However, it will be appreciated that useful markers need not be absolutely specific for the cell type of interest. For example, certain CD molecules are present on the cells of multiple different types of leukocytes. In general, a cell type specific marker for a particular cell type is expressed at levels at least 3 fold greater in that cell type than in a reference population of cells which may consist, for example, of a mixture containing cells from a plurality (e.g., 5-10 or more) of different tissues or organs in approximately equal amounts. More preferably the cell type specific marker is present at levels at least 4-5 fold, between 5-10 fold, or more than 10-fold greater than its average expression in a reference population. Preferably detection or measurement of a cell type specific marker makes it possible to distinguish the cell type or types of interest from cells of many, most, or all other types. In general, the presence and/or abundance of most markers may be determined using standard techniques such as Northern blotting, *in situ* hybridization, RT-PCR, sequencing, immunological methods such as immunoblotting, immunodetection, or fluorescence detection following staining with fluorescently labeled antibodies, oligonucleotide or cDNA microarray or membrane array, protein microarray analysis, mass spectrometry, etc.

"Ocular implant" refers to a drug delivery device that has appropriate structure, dimensions, shape, and/or configuration and is made of appropriate materials so that it may be placed in the eye without causing unacceptable interference with the physiology or functioning of the eye. Preferably placement of an ocular implant does not significantly disrupt vision. An ocular implant is typically a solid or semi-solid article of manufacture and is typically macroscopic, i.e., visible with the naked eye.

"Ocular neovascularization" (ONV) is used herein to refer to choroidal neovascularization or retinal neovascularization, or both.

"Polypeptide", as used herein, refers to a polymer of amino acids and/or amino acid analogs which may or may not be modified. Various amino acid analogs and modifications are described herein. A polypeptide may be cyclic or linear and may be branched or unbranched. The term "amino acid sequence" or "polypeptide sequence" as used herein can refer to the polypeptide material itself and is not restricted to the sequence information (i.e. the succession of letters or three letter codes chosen among the letters and codes used as abbreviations for amino acid names) that biochemically characterizes a polypeptide.

"Purified", as used herein, means separated from many other compounds or entities. A compound or entity may be partially purified, substantially purified, or pure. A compound or entity is considered pure when it is removed from substantially all other compounds or entities, i.e., is preferably at least about 90%, more preferably at least about 91%, 92%, 93%, 94%, 95%, 96%, 97%, 98%, 99%, or greater than 99% pure. A partially or substantially purified compound or entity may be removed from at least 50%, at least 60%, at least 70%, or at least 80% of the material with which it is naturally found, e.g., cellular material such as cellular proteins and/or nucleic acids.

"Retinal neovascularization" (RNV) refers to the abnormal development, proliferation, and/or growth of retinal blood vessels, e.g., on the retinal surface.

"Sequential administration" of two or more agents refers to administration of two or more agents to a subject such that the agents are not present together in the subject's body at greater than *de minimis* concentrations. Administration of the agents may, but need not, alternate. Each agent may be administered multiple times.

"Small molecule" refers to organic compounds, whether naturally-occurring or artificially created (*e.g*., via chemical synthesis) that have relatively low molecular weight and that are not proteins, polypeptides, or nucleic acids. Typically, small molecules have a molecular weight of less than about 1500 g/mol. Also, small molecules typically have multiple carbon-carbon bonds.

"Specific binding" generally refers to a physical association between a target polypeptide (or, more generally, a target molecule) and a binding molecule such as an antibody or ligand. The association is typically dependent upon the presence of a particular structural feature of the target such as an antigenic determinant or epitope recognized by the binding molecule. For example, if an antibody is specific for epitope A, the presence of a polypeptide containing epitope A or the presence of free unlabeled A in a reaction containing both free labeled A and the binding molecule that binds thereto, will reduce the amount of labeled A that binds to the binding molecule. It is to be understood that specificity need not be absolute but generally refers to the context in which the binding occurs. For example, it is well known in the art that numerous antibodies cross-react with other epitopes in addition to those present in the target molecule. Such cross-reactivity may be acceptable depending upon the application for which the antibody is to be used. One of ordinary skill in the art will be able to select antibodies or ligands having a sufficient degree of specificity to perform appropriately in any given application (e.g., for detection of a target molecule, for therapeutic purposes, etc). It is also to be understood that specificity may be evaluated in the context of additional factors such as the affinity of the binding molecule for the target versus the affinity of the binding molecule for other targets, e.g., competitors. If a binding molecule exhibits a high affinity for a target molecule that it is desired to detect and low affinity for nontarget molecules, the antibody will likely be an acceptable reagent. Once the specificity of a binding molecule is established in one or more contexts, it may be employed in other, preferably similar, contexts without necessarily re-evaluating its specificity. Binding of two or more molecules may be considered specific if the affinity (equilibrium dissociation constant, Kd) is 10⁻³ M or less, preferably 10⁻⁴ M or less, more preferably 10⁻⁵ M or less, e.g., 10⁻⁶ M or less, 10⁻⁷ Mor less, 10⁻⁸ M or less, or 10⁻⁹ M or less under the conditions tested, e.g., under physiological conditions.

"Subject", as used herein, refers to an individual to whom an agent is to be delivered, e.g., for experimental, diagnostic, and/or therapeutic purposes. Preferred subjects are mammals, particularly domesticated mammals (e.g., dogs, cats, etc.), primates, or humans.

"Significant sequence homology" as applied to an amino acid sequence means that the sequence displays at least approximately 20% identical or conservatively replaced amino acids, preferably at least approximately 30%, at least approximately 40%, at least approximately 50%, at least approximately 60% identical or conservatively replaced amino acids, desirably at least approximately 70% identical or conservatively replaced amino acids, more desirably at least approximately 80% identical or conservatively replaced amino acids, and most desirably at least approximately 90% amino acid identical or conservatively replaced amino acids relative to a reference sequence. When two or more sequences are compared, any of them may be considered the reference sequence. % identity can be calculated using a FASTA or BLASTP algorithm, using default parameters. A PAM250 or BLOSUM62 matrix may be used. For purposes of calculating % identical or conservatively replaced residues, a conservatively replaced residue is considered identical to the residue it replaces. Conservative replacements may be defined in accordance with Stryer, L,. Biochemistry, 3rd ed., 1988, according to which amino acids in the following groups possess similar features with respect to side chain properties such as charge, hydrophobicity, aromaticity, etc. (1) Aliphatic side chains: G, A, V, L, I; (2) Aromatic side chains: F, Y, W; (3) Sulfur-containing side chains: C, M; (4) Aliphatic hydroxyl side chains: S, T; (5) Basic side chains: K, R, H; (6) Acidic amino acids: D, E, N, Q; (7) Cyclic aliphatic side chain: P

"Substantial sequence homology" as applied to a sequence means that the sequence displays at least approximately 60% identity, desirably at least approximately 70% identity, more desirably at least approximately 80% identity, and most desirably at least approximately 90% identity relative to a reference sequence. When two or more sequences are compared, any of them may be considered the reference sequence. % identity can be calculated using a FASTA, BLASTN, or BLASTP algorithm, depending on whether amino acid or nucleotide sequences are being compared. Default parameters may be used. A PAM250 or BLOSUM62 matrix may be used.

A "sustained release formulation" is a composition of matter that comprises a therapeutic agent as one of its components and further comprises one or more additional components, elements, or structures effective to provide sustained release of the therapeutic agent, optionally in part as a consequence of the physical structure of the formulation. Sustained release is release or delivery that occurs either continuously or intermittently over a period of time e.g., at least 1, 2, 4, or 6 weeks, at least 1, 2, 3, 4, 6, 8, 10, 12, 15, 18, or 24 months, or longer.

"Treating" or "treatment of" as used herein, refers to providing any type of medical or surgical management to a subject. Treating can include, but is not limited to, administering a composition comprising a therapeutic agent to a subject. "Treating" includes any administration or application of a compound or composition of the invention to a subject for purposes such as curing, reversing, alleviating, reducing the severity of, inhibiting the progression of, or reducing the likelihood of a disease, disorder, or condition or one or more symptoms or manifestations of a disease, disorder or condition. A composition of this invention can be administered to a subject who has developed a macular degeneration related condition risk of developing an infection relative to a member of the general population. A composition of this invention can be administered to a subject who has developed an eye disorder such as exudative or non-exudative ARMD or diabetic retinopathy or is at increased risk of developing such a disorder relative to a member of the general population. A composition of this invention can be administered prophylactically, i.e., before development of any symptom or manifestation of the condition. Typically in this case the subject will be at risk of developing the condition.

The term "alkyl", as used herein, is to be taken to mean a straight, branched, or cyclic, substituted or unsubstituted alkyl chain of 1 to 6, preferably 1 to 4 carbons. Most preferably the alkyl group is a methyl group.

The term "acyl", as used herein, is to be taken to mean a substituted or unsubstituted acyl of 1 to 6, preferably 1 to 4 carbon atoms. Most preferably the acyl group is acetyl.

The term "aryl", as used herein, is to be understood to mean a substituted or unsubstituted homocyclic or heterocyclic aryl group, in which the ring preferably has 5 or 6 members.

As used herein, a "common" amino acid is an L-amino acid selected from the group consisting of glycine, leucine, isoleucine, valine, alanine, phenylalanine, tyrosine, tryptophan, aspartate, asparagine, glutamate, glutamine, cysteine, methionine, arginine, lysine, proline, serine, threonine and histidine. An "uncommon" amino acid includes, but is not restricted to, D-amino acids, homo-amino acids, N-alkyl amino acids, dehydroamino acids, aromatic amino acids (other than phenylalanine, tyrosine and tryptophan), ortho-, meta- or para-aminobenzoic acid, ornithine, citrulline, norleucine, γ-glutamic acid, aminobutyric acid and α,α-disubstituted amino acids.

### Detailed Description of Certain Embodiments of the Invention

**Overview**

The present invention provides compositions and methods for treatment and/or prevention of macular degeneration related conditions, ocular neovascularization, and/or ocular inflammation. As mentioned above, macular degeneration refers to a variety of degenerative conditions characterized by central visual loss due to deterioration of the macula. By far the most common of these conditions is age related macular degeneration (ARMD). Ambati, J., et al., Surv. Ophthalmol., 48(3): 257-293, 2003, and references therein, provide extensive information regarding the pathogenesis and current therapeutic strategies for ARMD.

Figures 1A and 1B show structures present in the anterior and posterior segments of the eye, including the retina, which contains the macula. Figures 1C-1E depict the outer layers of a normal eye (1C), an eye suffering from dry ARMD (1D), and an eye suffering from exudative (wet) ARMD (1E). The outer nuclear layer (ONL), contains nucleic of rod and cone photoreceptors. Each photoreceptor contains an innter segment (IS) and outer segment (OS), the latter of which contains the pigment rhodopsin, which initiaties the phototransduction cascade following exposure to light. The retinal pigment epithelial layer (RPE) lies below the photoreceptors and above Bruch's membrane, a layer of extracelluar matrix that separates the RPE from a networ of capillaries, the choriocapillaris (CC). Dry ARMD is characterized by separation of the RPE from BM, which is accompanied by RPE atrophy and apoptosis and loss of underlying choriocapillaris and overlying photoreceptors, resulting in areas of geographic atrophy which can eventually coalesce to form large patches. In exudative ARMD, new blood vessels grow from the choriocapillaris through Bruch's membrane and can extend into the RPE and photoreceptor cell layers (choroidal neovascularization). These blood vessels can bleed and leak fluid, frequently resulting in sudden visual loss due to events such as RPE and/or retinal detachment. Eventually a fibrovascular scar may form, leading to irreversible visual loss.

A variety of factors including oxidative stress, inflammation with a possible autoimmune component, genetic background (e.g., mutations), and environmental or behavioral features such as smoking and diet may contribute to the pathogenesis of ARMD in manners that are as yet poorly understood (Zarbin, MA, Arch Ophthalmol. 122:598-614, 2004). Regardless of the underlying etiology, the clinical hallmark of ARMD is the appearance of drusen, localized deposits of lipoproteinaceous material that accumulate in the space between the RPE and Bruch's membrane, which separates the RPE from the choroidal vessels (choriocapillaris). Drusen are typically the earliest clinical finding in ARMD. The existence of macular drusen is a strong risk factor for the development of both wet and dry forms of ARMD (Ambati, J., et al., Surv. Ophthalmol., 48(3): 257-293, 2003).

A large number of molecular components have been identified in drusen. These constituents include α1-antichymotrypsin, α1-antitrypsin, Alzheimer amyloid β peptide, advanced glycation end products, amyloid P component, apolipoproteins B and E, carbohydrate moieties recognized by various lectins, cholesterol esters, clusterin, complement factors, cluster differentiation antigen, complement receptor 1, factor X, heparan sulfate proteoglycan, human leukocyte antigen DR, immunoglobulin light chains, major histocompatibility complex class II antigens, membrane cofactor protein, peroxidized lipids, phospholipids and neutral lipids, tissue imhibitor of matrix metalloproeinases-3, transthyretin, ubiquitin, and vitronectin (Zarbin, 2004). A number of these components are also found in deposits associated with a variety of different diseases including atherosclerosis.

The complement system plays a crucial role in a number of physiological processes including the response to injury and defense against foreign entities such as infectious agents. The complement system is also known to play a role in a number of diseases (Makrides, SC, Pharm Rev., 50(1): 59-87, 1998). The complement system comprises more than 30 serum and cellular proteins that are involved in two major pathways, known as the classical and alternative pathways (*Kuby Immunology,* 2000).

The classical pathway is usually triggered by binding of a complex of antigen and IgM or IgG antibody to C1 (though certain other activators can also initiate the pathway). Activated C1 cleaves C4 and C2 to produce C4a and C4b, in addition to C2a and C2b. C4b and C2a combine to form C3 convertase, which cleaves C3 to form C3a and C3b. Binding of C3b to C3 convertase produces C5 convertase, which cleaves C5 into C5a and C5b. C3a, C4a, and C5a are anaphylotoxins and mediate multiple reactions in the acute inflammatory response. C3a and C5a are also chemotactic factors that attract immune system cells such as neutrophils. C3 and C5 convertase activity is controlled by a number of endogenous members of the Regulators of Complement Activation (RCA) family, also called Complement Control Protein (CCP) family, which includes complement receptor type 1 (CR1; C3b:C4b receptor), complement receptor type 2 (CR2), membrane cofactor protein (MCP; CD46), decay-accelerating factor (DAF), factor H (fH), and C4b-binding protein (C4bp). Makrides, 1998, and references therein describe the complement system and its components. RCA proteins are also described in U.S. Pat. No. 6,897,290.

The alternative pathway is initiated by microbial surfaces and various complex polysaccharides. In this pathway, C3b, resulting from cleavage of C3, which occurs spontaneously at a low level, binds to targets on cell surfaces and forms a complex with factor B, which is later cleaved by factor D, resulting in a C3 convertase. Cleavage of C3 and binding of another molecule of C3b to the C3 convertase gives rise to a C5 convertase. C3 and C5 convertases of this pathway are regulated by CR1, DAF, MCP, and fH. The mode of action of these proteins involves either decay accelerating activity (i.e., ability to dissociate convertases), ability to serve as cofactors in the degradation of C3b or C4b by factor I, or both.

The C5 convertases produced in both pathways cleave C5 to produce C5a and C5b. C5b then binds to C6, C7, and C8 to form C5b-8, which catalyzes polymerization of C9 to form the C5b-9 membrane attack complex (MAC). The MAC inserts itself into target cell membranes and causes cell lysis. Small amounts of MAC on the membrane of cells may have a variety of consequences other than cell death.

A third complement pathway, the lectin complement pathway is initiated by binding of mannose-binding lectin (MBL) and MBL-associated serine protease (MASP) to carbohydrates. In the human lectin pathway, MASP-1 and MASP-2 are involved in the proteolysis of C4, C2 and C3, leading to a C3 convertase described above.

Ocular inflammation can affect a large number of eye structures including the conjunctiva, cornea, episclera, sclera, uveal tract, retina, vasculature, optic nerve, and orbit. Evidence of ocular inflammation can include the presence of inflammation-associated cells such as white blood cells (e.g., neutrophils, macrophages) in the eye, the presence of endogenous inflammatory mediators known in the art, one or more symptoms such as eye pain, redness, light sensitivity, blurred vision and floaters, etc. Uveitis is a general term that refers to inflammation in the uvea of the eye, e.g., in any of the structures of the uvea, including the iris, ciliary body or choroid. Specific types of uveitis include iritis, iridocyclitis, cyclitis, pars planitis and choroiditis. Uveitis can arise from a number of different causes and is associated with a number of different diseases, including, but not limited to, rheumatic diseases such as rheumatic diseases (e.g., ankylosing spondylitis and juvenile rheumatoid arthritis), certain infectious diseases such as tuberculosis and syphilis, other conditions such as sarcoidosis, systemic lupus erythematosus, chemical injury, trauma, surgery, etc. Keratis refers to inflammation of the cornea. Keratitis has a diverse array of causes including bacterial, viral, or fungal infection, trauma, and allergic reaction. Amoebic infection of the cornea, e.g., caused by Acanthamoeba, is a particular problem for contact lens wearers. Scleritis refers to inflammation of the sclera. Uveitis, keratitis, and scleritis, and methods for their diagnosis are well known in the art. Symptoms of the various inflammatory conditions that affect the eye can include, but are not limited to, eye pain, redness, light sensitivity, tearing, blurred vision, floaters. Ocular inflammation of various types is well known to occur in association with a variety of local or systemic diseases, some of which are noted above. In some instances the cause may remain unknown.

**Methods of Treating or Preventing Macular Degeneration, ONV, and Ocular Inflammation**

The present invention is based in part on the recognition that certain compounds that are antagonists of G protein coupled receptors, particularly antagonists of the C5a receptor (C5aR), are particularly suitable as therapeutic agents for macular degeneration and related conditions, for diabetic retinopathy, and for ocular neovascularization due to either of these causes or others. As noted above, the compounds are referred to herein as GPCRAs. The invention is further based on the recognition that combinations comprising a GPCRA and a viral complement control protein are of particular value.

The ability of a number of cyclic or constrained acyclic compounds to bind to C5aR has been assessed. Certain of these compounds display the ability to modulate C5aR activity, e.g., by acting as agonists or antagonists. Compounds that have been tested are listed in Table 1. Exemplary methods for testing the binding of a compound to the C5aR and for assessing the ability of a compound to antagonize C5a are provided in U.S. Pat. No. 6,821,950 and in March, *supra.* For example, to assess binding of a test compound to C5a, fresh human polymorphonuclear leukocytes (PMNL), which express C5aR, are incubated in the presence of radiolabeled C5a and unlabeled test compound. The ability of the test compound to compete with C5a for binding to C5aR is determined. The ability of a test compound to act as a C5aR antagonist is assessed by incubating PMNLs in the presence of the test compound for a period of time, adding C5a, and then measuring release of myeloperoxidase.

Compounds that antagonize the activity of C5aR, e.g., by inhibiting binding of C5a to C5aR, are of use in the present invention for the treatment of a macular degeneration related condition or ocular neovascularization due to other causes.

In certain embodiments of the invention the compound is selected from the group consisting of SEQ ID NOs: 11, 12, 13, 14, 15, 16, 17, 18, 19, 20, 21, 22, 23, 24, 25, 26, 27 and 28, as described herein and in U.S. Pat. No. 6,821,950.

In certain embodiments of the invention the compound is SEQ ID NO: 46.

In certain embodiments of the invention the compound is selected from the group consisting of SEQ ID Nos: 29, 32, 46, 58, and 69.

In certain embodiments of the invention the compound is selected from the group consisting of SEQ ID Nos: 29, 30, 32, 33, 41, 46, 50, 52, 58, 69, and 74.

In certain embodiments of the invention the compound consists of at least 6 amino acid residues A-[B-C-D-E-F], wherein A is selected from the group consisting of: Suc-F (succinyl-phenyl alanine), Ac-F, and Hcin (hydrocinnamoyl); B is selected from the group consisting of: O (ornithine) (delta-N-Me)O and (delta-N-methylated ornithine); C is selected from the group consisting of: Hyp (4-trans-hydroxyproline) and P; D is selected from the group consisting of: dCha (d-cyclohexyalanine), dPhe (d-phenylalanine), and dhCha (d-homocyclohexylalanine); E is selected from the group consisting of W, F, and Bta (benzothiazolealanine), and 1Nal (1-naphthylalanine); and F is selected from the group consisting of: R and hArg (homoarginine). Square brackets indicate cyclic portion.

In certain embodiments of the invention the compound has a solubility of less than 5mg/ml, less than 1mg/ml, or less than 0.1mg/ml in an aqueous medium, e.g., water, physiological saline, etc. The solubility may be at least 0.01 mg/ml in water or saline. For example, the compound may have a solubility between 0.01 mg/ml and 5 mg/ml, e.g., between 0.01 and 1 mg/ml, or between 0.01 and .1 mg/ml in water or saline. In certain embodiments of the invention the compound has a solubility of greater than 0.1 mg/ml, greater than 1 mg/ml, greater than 5 mg/ml, or greater than 20 mg/ml in oils, lipids, hydrocarbons (such as olive oil, long or medium-chain triglycerides, hexane, etc.) The solubility may be between 0.1 mg/ml and 20 mg/ml, between 0.1 mg/ml and 50 mg/ml, etc. The compound can have any combination of the foregoing solubility characteristics. For example, the compound may have a solubility in water of less than 1 mg/ml and a solubility in oils, lipids, hydrocarbons of greater than 10 mg/ml. As is known in the art, "solubility" refers to the amount of a substance that dissolves in a given volume of solvent at a specified temperature and pH, e.g., to form a saturated solution. Solubility may be determined, for example, using the shake-flask solubility method (ASTM: E 1148-02, Standard Test Method for Measurements of Aqueous Solubility, Book of Standards Volume 11.05). Solubility may be determined at a pH between 3.0 and 9.0, e.g., between 4.0 and 8.0, between 5.0 and 8.0, between 6.0 and 8.0, e.g., between 6.5 and 7.6, e.g., between 6.8-7.4, e.g., 7.0, or any intervening value of the foregoing ranges. Solubility may be tested at a temperature of between 20 and 40°C, e.g., approximately 25-37°C, e.g., approximately 37°C, or any intervening value of the foregoing ranges. For example, solubility may be determined at approximately pH 7.0-7.4 and approximately 37°C. Without wishing to be bound by any theory, compounds having relatively poor solubility in water and relatively high solubility in oils, lipids, and/or hydrocarbons may have desirable characteristics for incorporation into an ocular implant, lipophilic emulsion, or polymeric delivery vehicle. Furthermore, low water and high lipophilicity may be desirable for good ocular tissue penetration. The invention provides an ocular implant, lipophilic emulsion, or polymeric delivery vehicle comprising a GPCRA having a solubility in water as described above and/or a solubility in oils, lipids, hydrocarbons as described above. The invention includes embodiments in which any of the compounds listed in Table 1 is modified to decrease solubility in water and/or to increase solubility in oils, lipids, or hydrocarbons. For example, a group such as an anthryl group may be added to any of the compounds listed in Table 1.

**Table 1**

| **SEQ ID NO:** | **Sequence** |
|---|---|
| 1 | (ISHKDMQLGR) |
| 2 | YSFKDMQLGR |
| 3 | YSFKDMPLaR (a = D-ala) |
| 4 | YSFKPMPLaR (a = D-ala) |
| 5 | RAARISLGPRahxYSFKPMPLaR (ahx = Acp; a = D-ala) |
| 6 | KYKHSVVKKahxYSFKPMPLaR (ahx = Acp; a = D-ala) |
| 7 | MeFKP(dCha) WR |
| 8 | MeFKP(dCha)WR- CONH₂ |
| 9 | MeFKP(dCha)WR |
| 10 | MeFKPLWR |
| 11 | AcF-[KPdChaWR] (R) |
| 12 | AcF-[OPdChaWR] (R) |
| 13 | F-[XPdChaWR] (S) |
| 14 | F-[XPdChaWR] (R) |
| 15 | F-[X²PdChaWR] (S) |
| 16 | F-[X²PdChaWR] (R) |
| 17 | AcF-[KPdChaWR] (S) |
| 18 | AcF-[OPdChaWR] (S) |
| 19 | [FWPdChaWR] |
| 20 | AcF-[KMdChaWR] |
| 21 | AcF-[KKdCha WR] |
| 22 | AcF-[XPdChaWR] |
| 23 | AcF-[X² PdChaWR] |
| 24 | AcKF-[OPdChaWR] |
| 25 | F-[OPdChaWR] |
| 26 | F-[KPdChaWR] |
| 27 | F-[OPdChaWR] |
| 28 | F-[KPdChaWR] |
| 29 | AcF-[OPdChaWR] |
| 30 | Mes-F-[OPdChaWR] |
| 31 | Tos-F-[OPdChaWR] |
| 32 | Suc-F-[OPdChaWR] |
| 33 | MeSuc-F-[OPdChaWR] |
| 34 | Ahx-F-[OPdChaWR] |
| 35 | Bn-F-[OPdChaWR] |
| 36 | Iso-F-[OPdChaWR] |
| 37 | Ac-G-[OPdChaWR] |
| 38 | Ac-f-[OPdChaWR] |
| 39 | Ac-Phg-[OPdChaWR] |
| 40 | Ac-hPhe-[OPdChaWR] |
| 41 | Ac-(o-fl)F-[OPdChaWR] |
| 42 | Ac-(m-fl)F-[OPdChaWR] |
| 43 | Ac-Y-[OPdChaWR] |
| 44 | H-[OPdChaWR] |
| 45 | Cin-[OPdChaWR] |
| 46 | HCin-[OPdChaWR] |
| 47 | AcF-[OV dChaWR] |
| 48 | AcF- [OFdChaWR] |
| 49 | AcF- [OIdChaWR] |
| 50 | AcF- [OHypdChaWR] |
| 51 | AcF- [OThpdChaWR] |
| 52 | AcF- [(δ-N-Me)OPdChaWR] |
| 53 | AcF- [OPGWR] |
| 54 | AcF- [OPdAlaWR] |
| 55 | AcF- [OPdVaIWR] |
| 56 | AcF- [OPdLeuWR] |
| 57 | AcF- [OPdNleWR] |
| 58 | AcF- [OPdPheWR] |
| 59 | AcF- [OP(N-Me)fWR] |
| 60 | AcF- [OPdhChaWR] |
| 61 | AcF- [OPdTyrWR] |
| 62 | AcF- [OPdArgWR] |
| 63 | AcF- [OPdTrpWR] |
| 64 | AcF- [OPdTicWR] |
| 65 | AcF- [OPdAicWR] |
| 66 | AcF- [OPdChaLR] |
| 69 | AcF- [OPdChaChaR] |
| 68 | AcF- [OPdChaHR] |
| 69 | AcF- [OPdChaFR] |
| 70 | AcF- [OPdChahFR] |
| 71 | AcF- [OPdCha(N-Me)FR] |
| 72 | AcF- [OPdChawR] |
| 73 | AcF- [OPdChaBtaR] |
| 74 | AcF- [OPdCha1Na1R] |
| 75 | AcF- [OPdCha2Na1R] |
| 76 | AcF- [OPdChaTicR] |
| 77 | AcF- [OPdChaFlaR] |
| 78 | AcF- [OPdChaWCit] |
| 79 | AcF- [OPdChaWhArg] |
| 80 | AcF- [OPdChaWK] |
| 81 | AcF- [OPfFR] |
| 82 | AcF- [OPf1Na1R] |
| 83 | AcF- [OPfYR] |
| 84 | AcF- [OPdChaF(pCl)F] |
| 85 | AcF- [OPdChaF(pNH₂)F] |
| 86 | AcF- [OPdChaFhF] |
| 87 | AcF- [OPdArgFR] |
| 88 | AcF- [OPGmbTyrR] |
| 89 | Ac-F-[OPdChaWR] |
| 90 | Ac-F-[OPdChaanthrylAR] |
| 91 | Ac-F-[OPdanthrylAWR] |
| 92 | Ac-F-[OanthrylAdChaWR] |
| 93 | Ac-anthrylA[OPdChaWR] |
| 94 | Fmoc-anthrylA-[OPdChaWR] |
| 95 | Fmoc-F-[OPdChaWR] |
| 96 | Fmoc-[OPdChaWR] |
| 97 | NH₂-[OPdChaWR] |
| 98 | F-[OPdChaWR] |
| 99 | Cin-[OPdChaWR] |
| 100 | HCin-[OPdChaWR] |

### Abbreviations

X = (CH₂)-NH₂; X² = (CH₂)₂-NH₂; Mes, mesyl; Tos, tosyl; Suc, succinyl; MeSuc, methyl succinyl; Ahx, aminohexanoyl; Bn, benzoyl; Iso, isovaleroyl; Phg, phenylglycine; hPhe or hF, homophenylalanine; (o-fl)F, ortho-fluoro phenylalanine; (m-fl)F, meta-fluoro phenylalanine; Cha, cyclohexylalanine; Cin, cinnamoyl; Hcin, hydrocinnamoyl; Hyp, 4-trans-hydroxyproline; Thp, 4-cis-thioproline; (δ-N-Me)O, δ-N-methylated ornithine; (N-Me)f, *N*-methyl-D-phenylalanine; hCha, homocyclohexylalanine; Tic, tetrahydroisoquinolone; Aic, aminoindanecarboxylic acid; hPhe, homophenylalanine; (N-Me)F, N-methyl-phenylalanine; Bta, benzothiazolealanine; 1nal, 1-Naphthylalanine; 2-Nal, 2- Naphthylalanine; Fla, fluorophenylalanine; Cit; citrulline; hArg, homoarginine; (pCl)F, *para-*chlorophenylalanine; (pNH₂)F, *para*-aminophenylalanine; mbTyr, metabenzyltryosine; anthrylA, anthrylalanine; 9-Fluorenylmethyloxycarbonyl.

Those of skill in the art will appreciate that many of the compounds encompassed by the structures herein may exhibit the phenomena of tautomerism, conformational isomerism, geometric isomerism and/or stereoisomerism. As the formulae drawings within this specification and claims can represent only one of the possible tautomeric, conformational isomeric, enantiomeric or geometric isomeric forms, it should be understood that the invention encompasses any tautomeric, conformational isomeric, enantiomeric and/or geometric isomeric forms of the compounds having one or more of the utilities described herein. As the nomenclature corresponds to the illustrated structural formulae, which represent only one of several possible tautomeric forms (or resonance structures) of the compounds, it will be understood that these references are for convenience only, and that any such references are not intended to limit the scope of the compounds described herein.

In addition, those of skill in the art also will recognize that the compounds of the invention may exist in many different protonation states, depending on, among other things, the pH of their environment. While the structural formulae provided herein depict the compounds in only one of several possible protonation states, it will be understood that these structures are illustrative only, and that the invention is not limited to any particular protonation state--any and all protonated forms of the compounds are intended to fall within the scope of the invention.

The compounds of the invention may, in certain embodiments, bear multiple positive or negative charges and may have appropriate counter ions associated therewith. Typically, the net charge of the compounds may be positive. The identity of the associated counter ions are may be governed the synthesis and/or isolation methods by which the compounds are obtained. Counter ions include, but are not limited to, chloride and other halides, acetate, trifluoroacetate, citrate, sulfate, phosphate, etc., and mixtures thereof. It will be understood that the identity of any associated counter ion is not a critical feature of the invention, and that the invention encompasses the compounds in association with any type of counter ion. Moreover, as the compounds can exists in a variety of different forms, the invention is intended to encompass not only forms that are in association with counter ions (e.g., dry salts), but also forms that are not in association with counter ions (e.g., aqueous or organic solutions).

**Compositions Comprising a GPCRA and an Additional Active Agent**

In certain embodiments which are not part of the present invention, a composition comprising a GPCR antagonist further comprises an additional active agent selected from the group consisting of: angiogenesis inhibitors, antiinflammatory agents, antiangiogenic steroid, complement inhibitors, and growth factors (e.g., growth factors for retinal pigment endothelial cells).

In certain embodiments of the invention the additional active agent is a complement inhibitor other than a GPCRA.

In certain embodiments of the invention the additional active agent is a complement inhibitor. The complement inhibitors may inhibit any complement component In certain embodiments of the invention the complement inhibitor is a viral complement control protein. Without wishing to be bound by any theory, the inventors propose that the combination of a GPCRA and (i) a complement inhibitor, e.g., a viral complement control protein (VCCP) such as VCP or SPICE; or (ii) an angiogenesis inhibitor such as a monoclonal antibody or aptamer that binds to VEGF (e.g., Avastin™, Lucentis™, or Macugen™); or (iii) a VCCP and an angiogenesis inhibitor, will prove unexpectedly beneficial. The invention specifically contemplates use of any of the agents described in U.S.S.N. 60/616,983, filed October 8, 2004, in U.S.S.N.11/247,886, filed Oct. 8, 2005, entitled VIRAL COMPLEMENT CONTROL PROTEINS FOR EYE DISORDERS, and/or in U.S.S.N. 60/751,771, filed Dec. 19, 2005, entitled VIRAL COMPLEMENT CONTROL PROTEINS FOR EYE DISORDERS CHARACTERIZED BY INFLAMMATION either in a composition together with a GPCRA or to be administered separately to a subject who also receives a GPCRA.

**Targeting Compounds to Sites in the Eye**

In addition to the methods described elsewhere herein, the invention provides a variety of compositions that facilitate targeting a GPCRA to a site in the eye. The invention provides a composition comprising (i) a GPCRA; and (ii) a moiety that binds to a component present in the eye of a subject at risk of or suffering from a macular degeneration related condition, ocular neovascularization, and/or ocular inflammation ("targeting moiety"). The composition can be used to treat or prevent a macular degeneration related condition, diabetic retinopathy, ocular neovascularization, or ocular inflammation. Preferably the moiety and the GPCRA are linked. The linkage can be covalent or noncovalent and can be direct or indirect in various embodiments of the invention. The moiety can be, for example, an antibody or ligand, as discussed below. According to certain embodiments of the invention the component is a cellular marker. In other embodiments of the invention the component is a drusen constituent. The cellular marker can be any marker that is expressed on or at the surface of a cell, preferably an endothelial cell or retinal pigment epithelial cell. In certain embodiments of the invention the cellular marker is a cell type specific marker.

In general, the component can be any molecule present on or at the surface of a cell or molecular entity (e.g., noncellular molecular entity). By "on or at the surface of the cell or molecular entity" is meant that the component is accessible to molecules present in the extracellular environment so that it can be recognized and bound by the moiety. The component may be entirely extracellular. The component may be inserted into the cell membrane. In certain embodiments of the invention the component may be partly or entirely within the membrane, in which case the entity must partially penetrate the membrane to gain access. In general, the component is not located in the cytoplasm of a cell. For example, protein kinase C (PKC) is not considered a cellular marker. As long as a sufficient portion of the component is exposed or accessible so that it can be recognized and bound, it will be said to be present on or at the surface. In preferred embodiments of the invention the component is a cellular marker, e.g., a cell type specific marker. Where the target is a molecular entity other than a cell, the component can be any chemical entity present on or at the surface of the molecule that is recognizable by an antibody or ligand. Drusen constituents such as those mentioned above are examples of noncellular molecular entities.

A number of cellular markers that are expressed on or at the surface of endothelial cells and can be used as targets to target a compound to endothelial cells in the eye (e.g., in the choroidal vasculature) are disclosed in U.S.S.N. 10/923,940. Tissue factor (TF), a molecule involved in hemostasis, is a preferred marker. Briefly, tissue factor is a cell membrane-bound glycoprotein (MW 46 kDa) and is a member of the class 2 cytokine receptor family. It is composed of a hydrophilic extracellular domain, a membrane-spanning hydrophobic domain, and a cytoplasmic tail of 21 residues, including a non-disulfide-linked cysteine. Upon exposure to blood, perivascular cell-bound TF binds to factor VII, a vitamin K-dependent serine protease. TF is expressed on endothelial cells lining the luminal surface of various forms of pathological neovasculature, including pathological vasculature associated with the exudative (wet) form of age-related macular degeneration and diabetic retinopathy but typically is not expressed (or is expressed at a much lower level) in normal vasculature, thus providing a specific and accessible therapeutic target. In those embodiments of the invention in which the cell type specific marker is TF, Factor VII (or a derivative thereof) is a suitable ligand that can be used to deliver a phagocytic marker to cells expressing TF. As mentioned above, TF binds to factor VII that is normally present in the blood. By linking a GPCRA to factor VII, the GPCRA is targeted to cells that express TF, e.g., endothelial cells in pathological neovasculature. Integrin alpha(v)beta(3) is another preferred marker.

A number of markers are expressed on or at the surface of retinal pigment epithelial cells. These include, but are not limited to, CD68 antigen (Elner SG, Exp Eye Res. 1992 Jul;55(1):21-8), claudin (Nishiyama K, et al., Anat Rec. 2002 Jul 1;267(3):196-203, the protein encoded by the RPE65 gene (Nicoletti A., et al., Invest Ophthalmol Vis Sci. 1998 Mar;39(3):637-44), CD45 and ICAM-1 (Limb, GA, et al., Curr Eye Res. 1997 Oct; 16(10):985-91). See also Chowers, I., et al., Studies on retinal and retinal pigment epithelial gene expression, Novartis Found Symp. 2004;255:131-45, 145-6, 177-8 for additional examples.

In general, a cell binding moiety is a molecule that comprises a portion that binds to a cellular marker. In certain preferred embodiments of the invention the cell binding moiety is linked to a GPCRA. In other embodiments the cell binding moiety comprises a portion that binds to another molecule to which a GPCRA is attached. Suitable cell binding moieties include antibodies that specifically bind to a cellular marker and ligands that specifically bind to a cellular marker. In general, the linkage between the cell binding moiety and the GPCRA can be covalent or noncovalent and can be direct or indirect in various embodiments of the invention. Similarly, a moiety that binds to a noncellular marker such as a drusen constituent may be linked to a GPCRA or to another molecule to which a GPCRA is attached.

In those embodiments of the invention in which the binding moiety is an antibody, the antibody may be any immunoglobulin or a derivative thereof which maintains binding ability, or any protein having a binding domain which is homologous or largely homologous to an immunoglobulin binding domain. Such proteins may be derived from natural sources, or partly or wholly synthetically produced (e.g., using recombinant DNA techniques, chemical synthesis, etc.). The antibody can be of any species, e.g., human, rodent, rabbit, goat, chicken, etc. The antibody may be a member of any immunoglobulin class, including any of the human classes: IgG, IgM, IgA, IgD, and IgE. In various embodiments of the invention the antibody may be a fragment of an antibody such as an Fab', F(ab')₂, scFv (single-chain variable) or other fragment that retains an antigen binding site, or a recombinantly produced scFv fragment, including recombinantly produced fragments. See, e.g., Allen, T., Nature Reviews Cancer, Vol.2, 750-765, 2002, and references therein. Monovalent, bivalent or multivalent antibodies can be used. The antibody may be a chimeric or "humanized" antibody in which, for example, a variable domain of rodent origin is fused to a constant domain of human origin, thus retaining the specificity of the rodent antibody. It is noted that the domain of human origin need not originate directly from a human in the sense that it is first synthesized in a human being. Instead, "human" domains may be generated in rodents whose genome incorporates human immunoglobulin genes. See, e.g., Vaughan, et al., (1998), Nature Biotechnology, 16: 535-539. The antibody may be partially or completely humanized. An antibody may be polyclonal or monoclonal, though for purposes of the present invention monoclonal antibodies are generally preferred. Preferably the antibody specifically binds to its target on the cell surface, e.g., to a cell-type specific marker. Methods for producing antibodies that specifically bind to virtually any molecule of interest are known in the art. For example, monoclonal or polyclonal antibodies can be purified from natural sources, e.g., from blood or ascites fluid of an animal that produces the antibody (e.g., following immunization with the molecule or an antigenic fragment thereof) or can be produced recombinantly, in cell culture.

In certain embodiments of the invention it is preferable to use F(ab')2 or F(ab') fragments rather than antibodies that contain an Fc portion since the Fc portion may have a pro-inflammatory effect or cause other undesirable effects. However, in certain embodiments of the invention it is preferred to use antibodies comprising an Fc domain. F(ab')₂ fragments can be generated, for example, through the use of an Immunopure F(ab')₂ Preparation Kit (Pierce) in which the antibodies are digested using immobilized pepsin and purified over an immobilized Protein A column. The digestion conditions (such as temperature and duration) may be optimized by one of ordinary skill in the art to obtain a good yield of F(ab')₂. The yield of F(ab')₂ resulting from the digestion can be monitored by standard protein gel electrophoresis. F(ab') can be obtained by papain digestion of antibodies, or by reducing the S-S bond in the F(ab')₂.

In various embodiments of the invention an appropriate ligand to which a GPCRA is linked can be any molecule that specifically binds to a target molecule (e.g., polypeptide or a portion thereof such as a carbohydrate moiety), through a mechanism other than an antigen-antibody interaction. For example, in various embodiments of the invention a ligand can be a polypeptide, peptide, nucleic acid (e.g., DNA or RNA), carbohydrate, lipid or phospholipid, or small molecule (e.g., an organic compound, whether naturally-occurring or artificially created that has relatively low molecular weight and is not a protein, polypeptide, nucleic acid, or lipid, typically with a molecular weight of less than about 1500 g/mol and typically having multiple carbon-carbon bonds).

Ligands may be naturally occurring or synthesized, including molecules whose structure has been invented by man. Examples of ligands include, but are not limited to, hormones, growth factors, oligo- or polysaccharide (and analogs), or neurotransmitters that bind to particular receptors. For example, Factor VII (FVII) is a ligand for TF. The activated form of FVII, FVIIa, has higher affinity for TF. Thus according to one embodiment of the invention a GPCRA is linked to FVIIa or a derivative thereof, e.g., a FVIIa peptide or inactivated form of FVIIa. The FVII derivative binds to TF, present on endothelial cells in ocular neovasculature, thereby providing an increased amount of GPCRA at the cell surface. Factor VII and peptides derived therefrom (e.g., Factor VIIa-derived fragments) of use in the invention are described in WO90/03390 and U.S. Pat. No. 5,962,418. Alternately, an inactive form of Factor VIIa is used. In some embodiments of the invention inactive FVII or inactive FVIIa is a derivative of FVII or FVIIa that is catalytically inactivated in the active site, e.g., by derivatization with an inhibitor. Examples of suitable inhibitors include peptide halomethyl ketones, e.g., peptide chloromethyl ketones (see, Williams et al., J. Biol. Chem. 264:7536-7540, 1989) or peptidyl halomethanes, e.g., peptidyl chloromethanes; azapeptides; acylating agents such as various guanidinobenzoate derivatives and the 3-alkoxy-4-chloroisocoumarins; sulphonyl fluorides such as phenylmethylsulphonylfluoride (PMSF); diisopropylfluorophosphate (DFP); tosylpropylchloromethyl ketone (TPCK); tosyllysylchloromethyl ketone (TLCK); nitrophenyl-sulphonates and related compounds; heterocyclic protease inhibitors such as isocoumarines, coumarins, organophosphor compound, and sulfonyl fluorides. Methods of using inhibitors to generate inactive FVII or FVIIa are known in the art. See, e.g., U.S. Pat. No. 5,817,788. In some embodiments FVII or FVIIa activity is inhibited by substitution, deletion, and/or insertion of one or more amino acids in FVII. Generally the substitution(s), insertion(s), and/or deletion(s) are made at or adjacent to a catalytic site residue. In certain embodiments, the alteration(s) is a substitution or deletion of Ser344, Asp242, and/or His193.

In one embodiment, the cell binding moiety is sialyl lewis x (sLex) or a derivative thereof. Slex is a tetrasaccharide that binds to selectins that are highly expressed on the surface of inflamed vascular endothelial cells, including those of experimental CNV (Shen,W.Y, et al., Br. J. Ophthalmol. 82, 1063-1071, 1998; Yeh,D.C., et al., Invest Ophthalmol. Vis. Sci. 45, 2368-2373, 2004). In one embodiment, particles (e.g., nanoparticles, microparticles, liposomes) containing one or more GPCRAs and, optionally, an additional active agent are conjugated with sLex. Methods for conjugating sLex to nanoparticles are known in the art (de la Fuente, J.M., et al., J. Am. Chem. Soc. 127, 6192-6197, 2005). In certain embodiments of the invention a linkable tetrasacccharide compound that includes the amino phenyl glycoside of Slex or a related analog is used. For example, sLex derivatives such as those described in U.S. Pub. No. 20040097403 may be used. In certain embodiments of the invention the sLex composition conjugated to the particles is multimeric.

In certain embodiments of the invention the GPCRA and the targeting moiety are linked by a cleavable linkage, e.g., a linkage that contains a bond that is susceptible to cleavage *in vivo* either through enzymatic or nonenzymatic means. Examples include disulfide bonds, ester linkages, amino acid sequences that contain cleavage sites for endogenous proteases, etc.

It will also be appreciated that fragments or variants of the above-mentioned polypeptide ligands differing in sequence from their naturally occurring counterparts but retaining the ability to bind to endothelial cells or retinal pigment epithelial cells can also be used. In certain embodiments of the invention a polypeptide ligand contains 5 or fewer amino acid differences, 10 or fewer amino acid differences, 25 or fewer amino acid differences, 50 or fewer amino acid differences, or 100 or fewer amino acid differences with respect to its naturally occurring counterpart. In certain embodiments of the invention the number of amino acid differences between a naturally occurring polypeptide ligand and a fragment or variant thereof for use in the invention is 5% or less, 10% or less, or 25% or less of the total number of amino acids in the naturally occurring polypeptide.

In certain embodiments of the invention a fragment or variant of a naturally occurring polypeptide ligand is at least 70% identical, at least 80% identical, at least 90% identical, at least 95% identical, over an amino acid portion that constitutes at least 10%, at least 20%, at least 30%, at least 40%, at least 50%, at least 60%, at least 70%, at least 80%, or at least 90%, or 100% of the length of the naturally occurring counterpart. For example, variant that exhibits at least 50%, at least 60%, at least 70%, at least 80%, at least 90%, or greater sequence identity, over the relevant portion of the sequence could be used, wherein %identity is determined as described above. The amino acid portion is preferably at least 20 amino acids in length, more preferably at least 50 amino acids in length. Alternately, a fragment or variant can display significant or, preferably, substantial homology to a naturally occurring counterpart. Generally a fragment or variant of a naturally occurring polypeptide ligand possesses sufficient structural similarity to its naturally occurring counterpart that it is recognized by an antibody (e.g., a polyclonal or monoclonal antibody) that recognizes the naturally occurring counterpart. Peptide ligands can be identified using phage display (Arap W, et al, Nature Medicine 8(2):121-7, 2002); Zurita AJ, et al., J. Control Release, 91(1-2):183-6, 2003; Pasqualini,R. & Ruoslahti,E. Nature 380, 364-366, 1996; Pasqualini,R., et al., Trends Mol. Med 8, 563-571, 2002).

In certain embodiments of the invention the ligand is an aptamer that binds to a cell type specific marker. In general, an aptamer is an oligonucleotide (e.g., DNA or RNA or) that binds to a particular protein. Aptamers are typically derived from an *in vitro* evolution process called SELEX, and methods for obtaining aptamers specific for a protein of interest are known in the art. See, e.g., Brody EN, Gold L. J Biotechnol. 2000 Mar;74(1):5-13. A pegylated anti-VEGF aptamer has shown some efficacy for CNV secondary to ARMD (The Eyetech Study Group, Opthalmology, 110(5), 2003).

Small molecules can also be used as ligands. Methods for identifing such ligands are known in the art. For example *in vitro* sreening of small molecule libraries, including combinatorial libraries, and computer-based screening, e.g., to identify small organic compounds that bind to concave surfaces (pockets) of proteins, can identify small molecule ligands for numerous proteins of interest (Huang, Z., Pharm. & Ther. 86: 201-215, 2000).

In certain embodiments of the invention binding moieties are not proteins or molecules that are typically used as carriers and conjugated to antigens for the purpose of raising antibodies. Examples are carrier proteins or molecules such as bovine serum albumin, keyhole limpet hemocyanin, bovine gamma globulin, and diphtheria toxin. In certain embodiments of the invention the cell binding moiety is not an Fc portion of an immunoglobulin molecule. However, in other embodiments any of these agents can be used as binding moieties.

Methods for covalently or noncovalently linking a GPCRA to a binding moiety are known in the art and are described in U.S.S.N. 10/923,940. General methods for conjugation and cross-linking are described in "Cross-Linking", Pierce Chemical Technical Library, available at the Web site having URL www.piercenet.com and originally published in the 1994-95 Pierce Catalog and references cited therein, in Wong SS, Chemistry of Protein Conjugation and Crosslinking, CRC Press Publishers, Boca Raton, 1991; and G. T. Hermanson, Bioconjugate Techniques, Academic Press, Inc., 1995. See also, Allen, T.M., Nature Reviews Cancer, 2, 750-763, 2002, which describes methods of making targeted therapeutic agents. For example, according to certain embodiments of the invention a bifunctional crosslinking reagent is used to couple a GPCRA with an antibody or ligand. In general, bifunctional crosslinking reagents contain two reactive groups, thereby providing a means of covalently linking two target groups. The reactive groups in a chemical crosslinking reagent typically belong to various classes including succinimidyl esters, maleimides, pyridyldisulfides, and iodoacetamides. Bifunctional chelating agents may also be used.

Targeted GPCRAs can be used for treatment of a number of conditions other than macular degeneration related conditions, diabetic retinopathy, or CNV. For such purposes the binding moiety need not bind to a site in the eye. In general, the binding moiety is selected to target the complement inhibiting protein to any site in the body at which complement inhibition is desired. For example, the compounds can be used to treat atherosclerosis, Alzheimer's disease, CNS injury (including spinal cord injury), transplant rejection, or any other disease in which complement activation plays a role (e.g., certain forms of glomerulonephritis, certain inflammatory conditions), etc. They can be used to prevent complement activation during cardiac bypass surgery or ischemia/reperfusion in myocardial infarction or stroke. Atherosclerotic plaques, organ transplants (e.g., xenotransplants, allotransplants, etc.) may be targeted. The targeted compositions can also be used *in vitro,* e.g., to treat platelets (which are considered cells for purposes of the invention) or other blood preparations in order to inhibit complement, or to treat organs prior to transplantation. Appropriate binding moieties, e.g., cell binding moieties or moieties that bind to a component in an atherosclerotic plaque, an Alzheimer's disease plaque (e.g., β-amyloid), etc. are used to target the GPCRA to the plaque. A Gal (1,3-Gal) epitope on the surface of a transplanted organ can be targeted.

**Modifications**

GPCRA linked to a binding moiety can be modified by addition of a molecule such as polyethylene glycol (PEG) or similar molecules to stabilize the compound or binding moiety, reduce its immunogenicity, increase its lifetime in the body, and/or increase its resistance to degradation. Methods for pegylation are well known in the art (Veronese,F.M. & Harris, Adv. Drug Deliv. Rev. 54, 453-456, 2002; Davis, F.F., Adv. Drug Deliv. Rev. 54,457-458 (2002; Hinds,K.D. & Kim,S.W. Adv. Drug Deliv. Rev. 54, 505-530 (2002; Roberts,M.J., Bentley,M.D. & Harris,J.M. Adv. Drug Deliv. Rev. 54, 459-476 (2002; Wang,Y.S. et al. Adv. Drug Deliv. Rev. 54, 547-570, 2002).

**Testing Therapeutic Potential in Animal Models**

A number of different animal models that attempt to replicate one or more features of macular degeneration, diabetic retinopathy, and/or choroidal neovascularization are known in the art. A compound of the invention can be administered in various doses to mice, rats, dogs, primates, etc. that have either spontaneous or macular degeneration and/or choroidal neovascularization or in which macular degeneration and/or choroidal neovascularization have been induced by a treatment. The ability of the compound to prevent or treat one or more indicia of macular degeneration (e.g. CNV, accumulation of lipofuscin in and/or drusen beneath the RPE, photoreceptor atrophy or hypertrophy, altered RPE pigmentation, photoreceptor loss, altered electroretinogram, etc.) is assessed. Visual examination, photography, histopathology, immunohistology, etc., can be used. Animal models for other ocular conditions are also known in the art.

Useful models include animals in which choroidal neovascularization is induced by laser treatment (Bora, P.S., et al., Proc. Natl. Acad Sci. 100(5): 2679-2684, 2003; Zacks, DN, et al., Invest Ophthalmol Vis Sci. 243(7):2384-91, 2002). Other models include animals that have been treated with a variety of agents such as lipid hydroperoxide (Tamai, K., et al., Exp Eye Res. 74(2):301-8, 2002), pellets comprising growth factors, etc. Animals genetically engineered to overexpress or underexpress one or more genes are also useful. For example, transgenic mice (mcd/mcd mice) that express a mutated form of cathepsin D that is enzymatically inactive display features associated with geographic atrophy (Rakoczy, PE, et al, Am. J. Path., 161(4), 1515-1524, 2002). Adeno-associated virus (AAV) mediated expression of vascular endothelial growth factor induces choroidal neovascularization in rats (Wang, F., et al., Invest Ophthalmol Vis Sci. 44(2):781-90, 2003). A preferred model is a transgenic mouse deficient in either monocyte chemoattractant protein (Ccl-2) or its cognate chemokine receptor (Ccr-2) (Ambati, J., et al., Nat Med. 9(11):1390-7, 2003; U.S.S.N. 10/685705-U.S. publication 20040177387). Aged mice with a defiency in either of these proteins exhibit a number of features of ARMD including accumulation of lipofuscin in and drusen beneath the RPE, photoreceptor atrophy, and CNV. Methods for testing the efficacy of a candidate agent using this mouse model are disclosed in U.S. publication no. 20040177387. In general, a candidate agent is administered to the mouse either before or after development of features of ARMD, and at least one eye is monitored for development or regression of drusen and/or lipofuscin accumulation therein, for affect of the candidate agent on Bruch's membrane, affect on retinal degeneration, and/or for affect on choroidal neovascularization. The candidate agent can be administered systemically or locally. The agent can be delivered orally, intravenously, intraperitoneally, intravitreously, transsclerally or topically. The agent can be delivered by intravitreal injection, by subretinal injection or instillation, transclerally, by sustained release implant, etc. The eye can be analyzed by ophthalmoscopy, angiography, histopathology or a combination thereof. Any of these methods can be used to assess efficacy of a candidate agent in any animal model. Models also exist for diabetic retinopathy. These examples are but a few of the model systems in which efficacy of the compounds of the invention can be assessed.

Compounds that show promising results in animal studies are tested in humans, e.g., using standard protocols and endpoints for clinical trials for therapies for conditions such as ARMD or diabetic retinopathy.

**Identifying Subjects and Assessing Response**

Methods for diagnosis of macular degeneration and CNV and for assessing response to therapy are known in the art. Any suitable tests and criteria can be used to identify a subject at risk of or suffering from a macular degeneration related condition, diabetic retinopathy, or choroidal neovascularization and/or to measure a response to therapy. Visual acuity can be measured using, for example, a Snellen chart, a Bailey-Lovie chart, a decimal progression chart, a Freiburg visual acuity test, a measurement of minimum angle of resolution (MAR) etc. Metamorphopsia (visual distortion) may be measured using an Amsler chart. Contrast sensitivity may be measured using a Pelli-Robson chart. Diagnostic studies include, but are not limited to, standard ophthalmologic examination of the fundus, stereo biomicroscopic examination of the macula, intravenous fundus fluorescein angiography, fundus photography, indocyanine green video-angiography, and optical coherence tomography. A subject displaying an abnormality on one or more of these diagnostic studies (e.g., a subject that falls outside a range that is considered normal for a healthy eye) may be treated in accordance with the present invention. Subjects may be classified as having early, intermediate, or advanced ARMD in accordance with the classification scheme used in the Age-Related Eye Diseases Study (AREDS), which is set forth in guidelines developed American Academy of Ophthalmology (American Academy of Opthalmology, Age Related Macular Degeneration Preferred Practice Pattern™, 2003; available for download at URL www.aao.org/aao/education/library/ppp/amd new.cfm). A subject falling into any of these categories may be treated in accordance with the present invention.

ARMD is known to have a genetic component, based on studies showing an increased incidence of ARMD in individuals with relatives suffering from ARMD (e.g., twin studies). Therefore, a subject may be considered at risk of developing ARMD if he or she has one or more close relatives (e.g., parent, grandparent, sibling, cousin, uncle, aunt), who has received a diagnosis of ARMD. A variety of specific genetic variants or mutations are believed to play a role in development of ARMD. Variations in the complement factor H *(CFH)* gene are associated with an increased risk for ARMD (Haines, JL, et al., Science, 308, 419-21, 2005; Klein, RJ, et al., Science, 308, 421-424, 2005). For example, a common missense variant, Y420H, in the complement factor H *(CFH)* gene increases the risk for advanced ARMD, soft drusen, geographic atrophy, and neovascular ARMD (Magnusson, KP, et al., PloS Medicine, 3(1), Jan. 2006). The gene encoding toll-like receptor 4 also contains susceptibility loci for development of ARMD (Zareparsi, S., et al., Hum Mol Genet., 14(11):1449-55, 2005). Genetic variation in the genes encoding factor B and complement component 2 are also of use in predicting risk of ARMD (Gold, B., et al., Nature Genetics, Advanced Online Publication March 5, 2006; doi:10.1038/ng 1750). Individuals who smoke and/or consume a high fat diet are also at increased risk. The incidence of ARMD increases with age. Therefore, an individual over approximately 50 years of age, generally at least 60 or at least 70 years of age may be considered at increased risk. An individual having drusen and one or more additional risk factors may be at particular risk for developing ARMD. An individual with multiple drusen, particularly if large and with indistinct borders, may be at particular risk. An individual with RPE hyperpigmentation or hypopigmentation or geographic atrophy may be at particular risk. Specific genetic mutations and variations are also associated with various macular degeneration related conditions that are less common than ARMD. A subject who has received a diagnosis of diabetes is at risk of developing diabetic retinopathy.

Response to therapy can be assessed by any of the methods mentioned above. Numerous studies have been conducted to assess the efficacy of a variety of different therapies in restoring vision, preventing visual loss, and/or resulting in improvement or slowing progression of ARMD or choroidal neovascularization as judged by diagnostic tests such as those described above. One of ordinary skill in the art will be able to select appropriate criteria by which to judge the efficacy of therapy.

An improvement in the condition of a subject's eye as a result of treatment with a compound or composition described herein may be, for example, any clinically significant improvement in a sign or symptom associated with a disorder. For example, the improvement may be an improvement in visual acuity (e.g., best corrected visual acuity) such as gaining 1, 2, 3, or more lines on an eye chart. The improvement may be a decrease in macular thickness, e.g., a decrease by at least 50 µm, at least 100 µm, at least 150 µm, etc. The improvement can be a decrease in the area of exudate evident in or on the retina.

The improvement can be an increase of at least 20%, 30%, 40%, 50%, 60%, 70%, 80%, 90%, 100%, 200%, 300%, or more, in any quantitative measure of the condition of the subject's eye, where an increase in the quantitative measure indicates improvement in the condition of the subject's eye. The improvement can be a decrease of at least 20%, 30%, 40%, 50%, 60%, 70%, 80%, 90%, or more, in any quantitative measure of the condition of the subject's eye, where a decrease in the quantitative measure indicates improvement in the condition of the subject's eye. If a scoring system is used as an indication of the condition of the subject's eye, (e.g., a scoring system including scores ranging from 1-3, 1-5, 1-10, etc.), the improvement may be, e.g., an increase of at least 1, 2, 3, or more units, where an increase in the score indicates improvement in the condition of the subject's eye. Alternately, if a scoring system in which a decrease in the score indicates an improvement in the condition of the subject's eye is used, the improvement may be a decrease of at least 1, 2, 3, or more units. One of ordinary skill in the art will appreciate that a variety of scoring systems may be used. For example, scores may be expressed in terms of units such as "+" or "-" rather than with integers. Of course it will be understood that individual responses will vary, and not all sujbects will respond. In animal studies and in clinical trials, changes in the condition of a subject's eye may be expressed in terms of an average or mean change in the population studied and/or using appropriate statistical tests.

**Therapeutic Applications**

The compositions of the invention can be administered to a subject to treat a macular degeneration related condition, diabetic retinopathy, retinopathy of prematurity, persistent hyperplastic vitreous syndrome, choroidal neovascularization, uveitis, etc. Ancillary therapies may also be used concurrently, prior to, or following treatment using the compositions and methods of the invention. Such therapies include, but are not limited to, administration of antioxidant vitamin and/or mineral therapy, photodynamic therapy (e.g., with verteporfin or other agents), administration of antiinflammatory agents, antiangiogenic therapy (e.g., angiogenesis inhibitors such as anecortave acetate or other angiostatic steroids; rhuRab V2 ranibizumab pegaptanib sodium anti-VEGF or anti-VEGFR siRNA, antisense RNA, or aptamer; or any other antiangiogenic agent including but not limited to a small molecule, siRNA, antisense RNA, or aptamer targeted to any proangiogenic gene), growth factor administration, implantation of cells (e.g., neural stem cells, RPE stems cells, RPE cells) into the eye, laser photocoagulation, radiation therapy, thermal therapy, and surgery (e.g., submacular surgery or macular translocation). In certain embodiments of the invention a growth factor for RPE cells is administered, e.g., REF-1/TFPI-2 (Tanaka, Y, et al., Invest Ophthalmol Vis Sci. 45(1):245-52, 2004).

It may be desirable to treat an eye that already suffers from ocular neovascularization (e.g., in a subject with diabetic retinopathy or ARMD) using photocoagulation or surgery and to also administer a composition of the invention to the subject to preserve vision in the other eye and/or prevent a recurrence of ONV in the eye treated with photocoagulation or surgery.

**Pharmaceutical Compositions and Delivery Vehicles and Methods**

Suitable preparations, e.g., substantially pure preparations of the compounds may be combined with pharmaceutically acceptable carriers, diluents, solvents, etc., to produce an appropriate pharmaceutical composition. The invention therefore provides a variety of pharmaceutically acceptable compositions for administration to a subject comprising (i) a GPCRA; and (ii) a pharmaceutically acceptable carrier, adjuvant, or vehicle. The invention further provides a pharmaceutically acceptable composition comprising (i) a GPCRA linked either directly or indirectly to a moiety that binds to a component present on or at the surface of a cell or noncellular molecular entity; and (ii) a pharmaceutically acceptable carrier, adjuvant, or vehicle. The moiety may be an antibody or ligand. The component may be a marker, such as a cell type specific marker.

In certain embodiments of the invention the pharmaceutical composition detectably inhibits neovascularization in an eye, following administration to a subject. In other words, administration of the compound measurably reduces neovascularization relative to the expected level in the absence of the composition. It is to be understood that the pharmaceutical compositions of the invention, when administered to a subject, are preferably administered for a time and in an amount sufficient to treat or prevent the disease or condition for whose treatment or prevention they are administered.

Further provided are pharmaceutically acceptable compositions comprising a pharmaceutically acceptable derivative (e.g., a prodrug) of any of the compounds of the invention, by which is meant any non-toxic salt, ester, salt of an ester or other derivative of a compound of this invention that, upon administration to a recipient, is capable of providing, either directly or indirectly, a compound of this invention or an inhibitorily active metabolite or residue thereof. As used herein, the term "inhibitorily active metabolite or residue thereof" means that a metabolite or residue thereof is also able to inhibit the activity of a GPCR, e.g., C5aR.

In various embodiments of the invention an effective amount of the pharmaceutical composition is administered to a subject by any suitable route of administration including, but not limited to, intravenous, intramuscular, by inhalation, by catheter, intraocularly, orally, rectally, intradermally, by application to the skin, etc.

Inventive compositions may be formulated for delivery by any available route including, but not limited to parenteral, oral, by inhalation to the lungs, nasal, bronchial, opthalmic, transdermal (topical), transmucosal, rectal, and vaginal routes. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered either locally to the eye or intravenously.

The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Solvents, dispersion media, coatings, antibacterial and antifungal agents, isotonic and absorption delaying agents, and the like, compatible with pharmaceutical administration may be included. Supplementary active compounds, e.g., compounds independently active against the disease or clinical condition to be treated, or compounds that enhance activity of a compound, can also be incorporated into the compositions. Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g., sodium and potassium), alkaline earth metal (e.g., magnesium), ammonium and N+(C1-4 alkyl)4 salts. This invention also envisions the quaternization of any basic nitrogen-containing groups of the compounds disclosed herein. Water or oil-soluble or dispersible products may be obtained by such quaternization.

A pharmaceutical composition is formulated to be compatible with its intended route of administration. Solutions or suspensions used for parenteral (e.g., intravenous), intramuscular, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment oftonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use typically include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ), phosphate buffered saline (PBS), or Ringer's solution.

Sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

In all cases, the composition should be sterile, if possible, and should be fluid to the extent that easy syringability exists.

Preferred pharmaceutical formulations are stable under the conditions of manufacture and storage and must be preserved against the contaminating action of microorganisms such as bacteria and fungi. In general, the relevant carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of injectable compositions can be brought about by including in the composition an agent which delays absorption, for example, aluminum monostearate and gelatin. Prolonged absorption of oral compositions can be achieved by various means including encapsulation.

Sterile injectable solutions can be prepared by incorporating the active compound in the required amount in an appropriate solvent with one or a combination of ingredients enumerated above, as required, followed by filtered sterilization. Preferably solutions for injection are free of endotoxin. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. Formulations for oral delivery may advantageously incorporate agents to improve stability within the gastrointestinal tract and/or to enhance absorption.

For administration by inhalation, the inventive compositions are preferably delivered in the form of an aerosol spray from a pressured container or dispenser which contains a suitable propellant, e.g., a gas such as carbon dioxide, or a nebulizer. Liquid or dry aerosol (e.g., dry powders, large porous particles, etc.) can be used. The present invention also contemplates delivery of compositions using a nasal spray.

For topical applications, the pharmaceutically acceptable compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For local delivery to the eye, e.g., as eyedrops, the pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

Methods of local administration include, e.g., choroidal injection, transscleral injection or placing a scleral patch, selective arterial catheterization, intraocular administration including transretinal, subconjunctival bulbar, intravitreous injection, suprachoroidal injection, subtenon injection, scleral pocket and scleral cutdown injection, by osmotic pump, etc. The agent can also be alternatively administered intravascularly, such as intravenously (IV) or intraarterially. In choroidal injection and scleral patching, the clinician uses a local approach to the eye after initiation of appropriate anesthesia, including painkillers and ophthalmoplegics. A needle containing the therapeutic compound is directed into the subject's choroid or sclera and inserted under sterile conditions. When the needle is properly positioned the compound is injected into either or both of the choroid or sclera. When using either of these methods, the clinician can choose a sustained release or longer acting formulation. Thus, the procedure can be repeated only every several months or several years, depending on the subject's tolerance of the treatment and response.

Intraocular administration of drugs intended for treatment of macular degeneration and other intraocular conditions is well known in the art. See, e.g., U.S. Patent Nos. 5,632,984 and 5,770,589. U.S. Patent No. 6,378,526 provides methods for intrascleral injection of a therapeutic or diagnostic material at a location overlying the retina, which provide a minimally invasive technique for delivering the agent to the posterior segment of the eye.

The invention provides sustained release compositions comprising a GPCRA that are suitable for administering the compound to the eye. A sustained release formulation of use in the present invention provides a therapeutic concentration of one or more active agent(s) within the eye or a portion or region thereof for a prolonged period of time. The period of time during which a therapeutic level of the active agent(s) is present can be, e.g., at least 1, 2, 4, or 6 weeks, at least 1, 2, 3, 4, 6, 8, 10, 12, 15, 18, 24 months, or longer. Release may begin immediately or shortly (e.g., within 24 hours) after administration of the sustained delivery formulation. Alternately, release may be delayed, e.g., it may commence at a time point at least 24 hours following administration. Without limitation, release may occur steadily or may occur intermittently (e.g., in bursts during which a substantial amount of the agent is released), or periods of steady release may alternate with bursts. In certain embodiments the therapeutic agent is released at controlled or predetermined rates when the sustained release formulation is placed in the eye. Such rates may range, for example, from about 0.003 micrograms/day to about 5000 micrograms/day, or between about .01 micrograms/day to about 5 micrograms/day, or between about .05 micrograms to about 1 microgram/day.

A sustained release formulation of use in the present invention typically comprises a therapeutic agent and an additional component, element, or structure that contributes to the sustained release properties of the formulation. The additional component, element, or structure that is effective to provide sustained release is referred to herein as a "drug delivery regulating component". Optionally the drug delivery regulating element is designed to provide control over the kinetics of release. It will be appreciated that the physical nature of the formulation, e.g., the shape and total surface area of any solid or semi-solid constituents, may contribute to its sustained release properties. As another example, tight compression of particles containing an active agent may result in release that takes place over a longer time period than if the particles were not compressed.

The drug delivery regulating component may comprise or consist of a polymer matrix that is physically associated with the therapeutic agent. For example, the therapeutic agent may be entrapped, embedded, or encapsulated by the polymer matrix. The polymeric matrix can comprise or consist essentially of biodegradable polymers, nonbiodegradable polymers, or can contain both biodegradable and nonbiodegradable polymers. A sustained release formulation can be in the form of an individual ocular implant, a plurality of nanoparticles, microparticles, or liposomes, a semi-solid or viscous material such as a gel, or combinations thereof, etc. The therapeutic agent may preferably be from about 1% to 90% by weight of the sustained release formulation. In certain embodiments of the invention the therapeutic agent is from 1-10% or 10%-20% by weight of the sustained release formulation. In certain embodiments of the invention the therapeutic agent is from about 20% to about 80% by weight of the of the sustained release formulation. In certain embodiments, the therapeutic agent comprises about 40% by weight of the sustained release formulation. (e.g., 30%-50%).

A number of polymeric delivery vehicles for providing sustained release have been used in an ocular context and can be used to administer the compositions of the invention. Various polymers, e.g., biocompatible polymers, which may be biodegradable, can be used. The polymers may be homopolymers, copolymers (including block copolymers), straight, branched-chain, or cross-linked. Useful polymers include, but are not limited to, poly-lactic acid (PLA), poly-glycolic acid (PGA), poly-lactide-co-glycolide (PLGA), poly(phosphazine), poly (phosphate ester), polycaprolactones, polyanhydrides, ethylene vinyl acetate, polyorthoesters, polyethers, and poly (beta amino esters). Peptides, proteins such as collagen or albumin, polysaccharides such as chitosan, alginate, hyaluronic acid (or derivatives of any of these) and dendrimers (e.g., PAMAM dendrimers) are also of use. Methods for preparation of such formulations will be apparent to those skilled in the art. Certain of the materials can also be obtained commercially, e.g., from Alza Corporation and Nova Pharmaceuticals, Inc. Any of these polymers, or combinations thereof, can be used in various embodiments of the invention.

Additional exemplary polymers include cellulose derivatives such as carboxymethylcellulose, polycarbamates or polyureas, cross-linked poly(vinyl acetate) and the like, ethylene-vinyl ester copolymers having an ester content of 4 to 80% such as ethylene-vinyl acetate (EVA) copolymer, ethylene-vinyl hexanoate copolymer, ethylene-vinyl propionate copolymer, ethylene-vinyl butyrate copolymer, ethylene-vinyl pentantoate copolymer, ethylene-vinyl trimethyl acetate copolymer, ethylene-vinyl diethyl acetate copolymer, ethylene-vinyl 3-methyl butanoate copolymer, ethylene-vinyl 3-3-dimethyl butanoate copolymer, and ethylene-vinyl benzoate copolymer, or mixtures thereof. In certain embodiments a substantially nonbiodegradable polymer is used.

Poly(ortho esters) have been introduced into the eye and demonstrated favorable properties for sustained release ocular drug delivery (Einmahl, S., Invest. Ophthalmo/. Vis. Sci., 43(5), 2002). Polylactide particles have been used to target an agent to the retina and RPE following intravitreous injection of a suspension of such particles (Bourges, J-L, et al, Invest. Ophthalmol. Vis. Sci., 44(8), 2003).

Sustained release formulations including various ocular implants and other ocular drug delivery systems that are of use in various embodiments of the invention are described, for example, in U.S. Patent Nos. 6,692,759; 6,331,313; 5,869,079; 5,824,072; and U.S.S.N. 10/918,597 (Pub. No. 20050048099); 10/837,357 (Pub. No. 20050244469); 11/092,122 (Pub. No. 20050244472) and 11/116,698 (Pub. No. 20050281861) as well as a number of other patents and publications referenced in the foregoing, all of which are incorporated herein by reference.

A method of making a sustained release formulation involves combining or mixing the therapeutic agent with a polymeric component to form a mixture. The mixture may then be extruded, compressed, molded, etc., to form a single composition. Optionally, heat and/or pressure can be used. The single composition may then be processed to form individual implants or particles suitable for placement in an eye of a patient. Additional methods for incorporating therapeutically active agents into polymeric matrices are known in the art. The polymeric matrix can be formed into various shapes such as rods, disks, wafers, etc., which may have a range of different dimensions (e.g., length, width, etc.) and volumes. Exemplary shapes include spherical, cylindrical, helical, coil-shaped or helical, screw-shaped, cubical, conical, ellipsoidical, biconvex, hemispherical or near-hemispherical etc.

In certain embodiments of the invention an ocular implant is so dimensioned and shaped that it fits within the hollow shaft of an injection needle, e.g., a 22, 25, 27, 30, 33, or 35 gauge needle (or needle of any gauge ranging between 22 and 35). Exemplary and nonlimiting dimensions for a cylindrical implant may be about 0.5 to 8 millimeters in length and about 0.1 to 2 millimeters in diameter, e.g., about 0.75 mm to about 1.5 mm in diameter. Implants having other shapes, e.g., other rodlike structures with cross-sections that are rectangular or square in cross-section may have a cross-section in which the two points most distant from each other are separated by at most 0.1 mm to 1 mm. In particular embodiments the intraocular implant may have a length or other longest dimension of between about 5 microns and about 2 mm, or between about 10 microns and about 1 mm for administration with a needle. Alternately, the length or other longest dimension is greater than 1 mm, or greater than 2 mm, such as 3 mm or up to 10 mm. The vitreous chamber in humans is able to accommodate relatively large implants of varying geometries, having lengths of, for example, 1 to 10 mm.

In certain embodiments of the invention the implants may also be at least somewhat flexible, which may facilitate both insertion of the implant in the eye, e.g., in the vitreous, and/or may facilitate accommodation of the implant. The total weight of the implant may be about 250-5000 micrograms, e.g., about 500-1000 micrograms. For example, an implant may weigh about 500 micrograms or about 1000 micrograms. Larger implants may also be formed and further processed before administration to an eye. In addition, larger implants may be desirable where relatively greater amounts of a therapeutic agent are provided in the implant, as used. In various embodiments the ocular implant contains, e.g., between 10 micrograms and 1000 micrograms of an active agent, e.g., between 100 and 500 micrograms, e.g., about 250 micrograms.

The active agent may diffuse out of an implant or may be released as the implant degrades. In one embodiment the sustained release formulation is a biocompatible ocular implant comprising a substantially impermeable polymeric outer layer covering a core which comprises the drug to be delivered, wherein said outer layer has one or more orifices, by which is meant one or more openings in the outer layer through which, when the device is in use, body fluids can enter the device and the drug contained in the device (e.g., dissolved, encapsulated, or entrapped within the device) can migrate out of the device. In certain embodiments the orifices in total have a surface area of less than 10 percent of the total surface area of the device. In certain embodiments of the invention the ocular implant comprises an outer coating layer that is permeable to the therapeutic agent, allowing its slow diffusion out of the implant. The composition, structure, and/ or thickness of the coating layer may be selected to provide a particular permeability and diffusion rate.

A drug can be contained in an ocular implant as a dry powder, particles, granules, or as a compressed solid. The drug may also be present as a solution or be dispersed in a polymer matrix. Ocular implants, may be have the active agent or agents homogenously distributed through the polymeric matrix, e.g., they may be monolithic. In other embodiments the active agent(s) are heterogeneously distributed in the polymeric matrix. For example, discrete regions of the implant may contain solid particles of an active agent, or a reservoir of active agent may be encapsulated by the polymeric matrix. The therapeutic agent(s) may be distributed in a non-homogenous pattern in the matrix. For example, an implant may include a portion that has a greater concentration of the therapeutic agent relative to a second portion of the implant. Multilayered structures, with the layers having different compositions and may have different physical characteristics such as density or porosity are another possibility. For example, the layers may contain different therapeutic agents or combinations thereof. In another embodiment, layers that are relatively resistant to degradation are interspersed with layers that degrade more rapidly.

The biodegradable polymeric materials which are included to form the matrix may be subject to enzymatic or hydrolytic instability. Water soluble polymers may be cross-linked with hydrolytic or biodegradable unstable cross-links to provide useful water insoluble polymers. The degree of stability can vary widely, depending, for example, upon the choice of monomer, whether a homopolymer or copolymer or mixture, is employed, and whether the polymer includes terminal acid groups. The biodegradation of the polymer and hence the extended release profile of the sustained release formulation may also influenced by the relative average molecular weight of the polymeric materials employed. Different molecular weights of the same or different polymeric materials may be included in the formulations to modulate the release profile. For example, the average molecular weight of the polymer may range from about 5 to about 500 kDa, e.g., from about 10 to 100 kDa, or from about 15 to 50 kDa.

Nanoparticles or microparticles can be made using any method known in the art including, but not limited to, spray drying, phase separation, single and double emulsion, solvent evaporation, solvent extraction, and simple and complex coacervation. Particulate polymeric compositions can also be made using granulation, extrusion, and/or spheronization. A composition can contain nanoparticles or microparticles having different compositions and/or properties. Exemplary materials from which nanoparticles or microparticles can be made include the various polymers described above.

The conditions used in preparing the particles may be altered to yield particles of a desired size or property (e.g., hydrophobicity, hydrophilicity, external morphology, "stickiness", shape, etc.). The method of preparing the particle and the conditions (e.g., solvent, temperature, concentration, air flow rate, etc.) used may also depend on the therapeutic agent and/or the composition of the polymer matrix.

Microparticles and nanoparticles of use in the invention can have a range of dimensions. Generally, a microparticle will have a diameter of 500 microns or less, e.g., between 1 and 500 microns, between 50 and 500 microns, between 100 and 250 microns, between 20 and 50 microns, between 1 and 20 microns, between 1 and 10 microns, etc., and a nanoparticle will have a diameter of less than 1 micron, e.g., between 10 nm and 100 nm, between 100 nm and 250 nm, between 100 nm and 500 nm, between 250 nm and 500 nm, between 250 nm and 750 nm, between 500 nm and 750 micron. If the particles prepared by any of the above methods have a size range outside of the desired range, the particles can be sized, for example, using a sieve. Particles can be substantially uniform in size (e.g., diameter) or shape or may be heterogeneous in size and/or shape. They may be substantially spherical or may have other shapes, in which case the relevant dimension will be the longest straight dimension rather than the diameter.

In certain embodiments of the invention a sustained release formulation comprises a therapeutic agent and a gel-forming material. In accordance with certain embodiments of the invention, a solution containing the soluble gel-forming material and a therapeutic agent is prepared by combining the soluble gel-forming material and therapeutic agent in solution using any suitable method, e.g., by adding the therapeutic agent to a solution containing soluble collagen. The composition is delivered locally to an appropriate location in the eye of a subject.. The solution rapidly forms a gel at or close to of the site of administration. The therapeutic agent is entrapped within the gel. The therapeutic agent diffuses out of the gel or is released as the gel degrades over time, thereby providing a continuous supply of the agent to tissues and structures that are either in direct physical contact with the gel or located nearby. In certain embodiments the solution is administered behind the sclera of the eye. Delivery can be accomplished by injection (e.g., using a 25, 27, or 30 gauge needle or the like), by catheter, etc.

In one embodiment, soluble collagen is used as the gel-forming material. The collagen is initially soluble, e.g., in an aqueous medium, and forms a solution that has a low viscosity but is capable of rapid formation of a gel under appropriate conditions, e.g., conditions encountered upon administration to a mammalian subject. A variety of different collagen preparations can be used in the present invention provided that the collagen is initially soluble and is capable of rapidly forming a gel under appropriate conditions. Suitable collagen preparations, and methods for their manufacture, are described, e.g., in U.S. Pat. Nos. 5,492,135; 5,861,486; 6,197,934; 6,204,365; and WO 00/47130, but the invention is not limited to such preparations or methods. These collagens are prepared in soluble form and rapidly form a gel upon exposure to physiological fluids or other fluids having suitable concentration of ions. In accordance with the present invention, injecting or otherwise introducing the collagen solution to the eye or near the eye results in gel formation, presumably induced by contact with physiological fluids. However it is noted that the invention is in no way limited by the mechanism by which gel formation occurs. In addition, as noted above, the gel can be formed *in vitro* and then implanted at an appropriate location.

Other gel-forming materials of use in the invention include, but are not limited to, hyaluronic acid and modified forms thereof, polysaccharides such as alginate and modified forms thereof, self-assembling peptides, etc. See, e.g., U.S. Pat. Nos. 6,129,761 for further description of alginate and modified forms thereof, hyaluronic acid and modified forms thereof, and additional examples of soluble gel-forming materials that are of use in various embodiments of the present invention. As described therein, other polymeric hydrogel precursors include polyethylene oxide-polypropylene glycol block copolymers such as Pluronics™ or Tetronics™ which are crosslinked by hydrogen bonding and/or by a temperature change, as described in Steinleitner et al., Obstetrics & Gynecology, 77:48-52 (1991); and Steinleitner et al., Fertility and Sterility, 57:305-308 (1992). Other materials which may be utilized include proteins such as fibrin or gelatin. Polymer mixtures also may be utilized. For example, a mixture of polyethylene oxide and polyacrylic acid which gels by hydrogen bonding upon mixing may be utilized.

Typically a gel-forming material of use in the invention is capable of being at least partly dissolved, or in certain embodiments of the invention substantially or fully dissolved, e.g., in an aqueous medium. For example, at least 50%, at least 60%, at least 70%, at least 80%, at least 90%, at least 95%, or more, by weight, of the gel-forming material present in a gel-forming composition may be dissolved. In certain embodiments essentially 100% of the material is dissolved. It will be appreciated that the aqueous medium can contain one or more liquids in addition to water, e.g., various alcohols. In general, at least 10%, 20%, 30%, 40%, 50%, 60%, 70%, 80%, 90%, 95%, or 100% of the liquid present in the medium is water.

Covalently crosslinkable hydrogel precursors also are useful. For example, a water soluble polyamine, such as chitosan, can be cross-linked with a water soluble diisothiocyanate, such as polyethylene glycol diisothiocyanate. The isothiocyanates will react with the amines to form a chemically crosslinked gel. Aldehyde reactions with amines, e.g., with polyethylene glycol dialdehyde also may be utilized. A hydroxylated water soluble polymer also may be utilized.

In certain embodiments of the invention a therapeutic agent is covalently or noncovalently attached to a drug delivery regulating component such as a polymer via a linking moiety. The linking moiety is cleaved to release the therapeutic agent from the drug delivery regulating component to provide sustained release. For example, the linking moiety may be a peptide containing a site that is cleaved by an endogenous enzyme such as a protease, or the linking moiety may contain a labile or hydrolyzable bond, e.g., a disulfide bond, ester moiety, etc.

Cells that express a therapeutic agent that is a biological macromolecule such as a protein or RNAi agent can be implanted into the eye and are of use in certain embodiments of the invention to provide sustained release. U.S. Patent No. 6,436,427 provides a method for delivering biologically active molecules to the eye by implanting biocompatible capsules containing a cellular source of the biologically active molecule.

In certain embodiments of the invention the sustained release formulation comprises liposomes. For example, a liposomal suspension can be administered. Liposomes can be prepared according to methods known to those skilled in the art, for example, as described in U.S. Patent No. 4,522,811 and other references listed herein. Liposomes, including targeted liposomes (e.g., antibody targeted liposomes) and pegylated liposomes have been described (Hansen CB, et al., Biochim Biophys Acta. 1239(2):133-44,1995; Torchilin VP, et al., Biochim Biophys Acta, 1511(2):397-411, 2001; Ishida T, et al., FEBS Lett. 460(1):129-33, 1999).

One of ordinary skill in the art will appreciate that the materials and methods selected for preparation of a sustained release formulation, implant, etc., should be such as to retain activity of the compound. For example, it may be desirable to avoid excessive heating of certain agents such as polypeptides, which could lead to denaturation and loss of activity. Furthermore, it will be appreciated that a sustained release formulation may contain a variety of additional components that lack therapeutic activity and that may or may not contribute to the sustained release features of the formulation. Examples include plasticizing agents, solubilizing agents, solubility decreasing agents, and dispersing agents (see U.S. Pat. No. 6,331,313), provided that such components are compatible with administration to the eye under the conditions used. For example, a sustained release formulation may include a β-cyclodextrin, which is effective in enhancing the solubility of the therapeutic agent. The β-cyclodextrin may be provided in an amount from about 0.5% (w/w) to about 25% (w/w) of the implant. In certain implants, the β-cyclodextrin is provided in an amount from about 5% (w/w) to about 15% (w/w) of the formulation. Other formulations include a gamma-cyclodextrin, and/or cyclodextrin derivatives.

A sustained release formulation herein may include an excipient component, such as effective amounts of buffering agents, preservatives and the like. Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents are advantageously present in amounts sufficient to maintain a pH of the system of between about 2 to about 9 and more preferably about 4 to about 8. As such the buffering agent may be as much as about 5% by weight of the total system. Suitable water soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from 0.001 to about 5% by weight and preferably 0.01 to about 2% by weight. These agents may also be used in certain of the liquid compositions described herein.

In some embodiments of the invention the sustained release formulation comprises an agent that enhances uptake of the therapeutic agent by cells, enhances bioavailability of the agent at its site of action, or otherwise enhances activity of the therapeutic agent. For example, a variety of delivery vehicles that enhance uptake and/or activity of RNAi agents such as siRNAs are known in the art and may be included in the sustained release formulation.

One aspect of the invention is the recognition that certain compounds of the invention have particularly desirable properties for use in an ocular implant. The invention provides a method of selecting a GPCRA or a polymer for use in an ocular implant comprising: preparing a composition comprising a polymeric matrix containing the polymer and a GPCRA; contacting the composition with a fluid; and measuring release of the GPCRA into the fluid over time. The fluid may be any fluid of choice, e.g., water, saline, plasma, serum, cell culture medium, etc. The test may be performed at a variety of temperatures, e.g., a temperature between 20°C and 40°C, e.g., 25°C or 37°C. The activity of the released GPCRA can be determined, e.g., by measuring its ability to act as a C5aR antagonist. A USP approved method for dissolution or release test can be used to measure the rate of release (USP 23; NF 18 (1995) pp. 1790-1798). Instead of measuring released GPCRA, the amount of GPCRA remaining in the polymeric matrix can be determined. Methods include weighing the polymeric matrix (accounting for any decrease in weight due to degradation of the matrix). The polymeric matrix can comprise any one or more polymers including, but not limited to, those disclosed herein. Any GPCRA can be tested. Desired proportions of GPCRA or other therapeutic agent, polymer, and any other modifiers may be empirically determined by formulating multiple compositions with varying proportions of such ingredients. In one embodiment a GPCRA and a polymer are selected, wherein a composition comprising the GPCRA and the polymer releases less than 75% of the GPCRA during the first 1, 2, 3, 4, 5, 6, 7, 8, 9, 10, 11, or 12 weeks of the test. In one embodiment a GPCRA and a polymer are selected, wherein a composition comprising the GPCRA and the polymer releases less than 50% of the GPCRA during the first 1, 2, 3, 4, S, 6, 7, 8, 9, 10, 11, or 12 weeks of the test. In one embodiment a GPCRA and a polymer are selected, wherein a composition comprising the GPCRA and the polymer releases less than 25% of the GPCRA during the first 1, 2, 3,4, 5, 6, 7, 8, 9, 10, 11, or 12 weeks of the test. The invention provides a composition comprising a GPCRA and a polymer, wherein the composition releases less than 75% of the GPCRA during the first 1, 2, 3, 4, S, 6, 7, 8, 9, 10, 11, or 12 weeks of incubation in a fluid of choice. The invention additionally provides a composition comprising a GPCRA and a polymer, wherein the composition releases less than 50% of the GPCRA during the first 1, 2, 3, 4, 5, 6, 7, 8, 9, 10, 11, or 12 weeks of incubation in a fluid of choice. The invention further provides a composition comprising a GPCRA and a polymer, wherein the composition releases less than 25% of the GPCRA during the first 1, 2, 3, 4, S, 6, 7, 8, 9, 10, 11, or 12 weeks of incubation in a fluid of choice. The release may occur at a temperature between about 20°C and 40°C, e.g., 25°C or 37°C. The compositions may be in the form of implants. The implants can also be tested *in vivo.*

Included within the scope of the term "sustained release formulation of a therapeutic agent" are devices or "chips" that include one or more reservoirs containing the agent and that release the agent or a portion thereof from the one or more reservoirs into the surrounding environment (see, e.g., U.S. Pat. Nos. 5,797,898 and 6,976,982). Release may occur through a variety of means. For example, the reservoirs may have a biodegradable cap that is impermeable to the agent and degrades over time, so that the therapeutic agent is released once the cap is degraded. Caps of differing thickness will cause release to occur at different times. Mechanical, electrical, or other means may be used to release the agent from a reservoir, optionally using external control means to regulate such release. Release can occur at predetermined times and/or in predetermined amounts. The device may be programmable.

The pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In addition to the agents described above, in certain embodiments of the invention, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polyethers, and polylactic acid. Methods for preparation of such formulations will be apparent to those skilled in the art. Certain of the materials can also be obtained commercially from Alza Corporation and Nova Pharmaceuticals, Inc. Liposomal suspensions can also be used as pharmaceutically acceptable carriers. These can be prepared according to methods known to those skilled in the art, for example, as described in U.S. Patent No. 4,522,811 and other references listed herein. Liposomes, including targeted liposomes (e.g., antibody targeted liposomes) and pegylated liposomes have been described (Hansen CB, et al., Biochim Biophys Acta. 1239(2):133-44,1995; Torchilin VP, et al., Biochim Biophys Acta, 1511(2):397-411, 2001; Ishida T, et al., FEBS Lett. 460(1):129-33, 1999). One of ordinary skill in the art will appreciate that the materials and methods selected for preparation of a controlled release formulation, implant, etc., should be such as to retain activity of the compound. For example, it may be desirable to avoid excessive heating of polypeptides, which could lead to denaturation and loss of activity.

It is typically advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ ED₅₀. Compounds which exhibit high therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma can be measured, for example, by high performance liquid chromatography.

A therapeutically effective amount of a pharmaceutical composition typically ranges from about 0.001 to 100 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The pharmaceutical composition can be administered at various intervals and over different periods of time as required, e.g., multiple times per day, daily, every other day, once a week for between about 1 to 10 weeks, between 2 to 8 weeks, between about 3 to 7 weeks, about 4, 5, or 6 weeks, etc. The skilled artisan will appreciate that certain factors can influence the dosage and timing required to effectively treat a subject, including but not limited to the severity of the disease or disorder, previous treatments, the general health and/or age of the subject, and other diseases present. Generally, treatment of a subject with an inventive composition can include a single treatment or, in many cases, can include a series of treatments.

Exemplary doses include milligram or microgram amounts of the inventive compunds per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram.) For local administration doses much smaller than these may be used. Exemplary doses include between 1 and 1000 micrograms of an active agent, e.g., between 10 and 500 micrograms, e.g., between 10 and 100 micrograms.

It is furthermore understood that appropriate doses depend upon the potency of the agent, and may optionally be tailored to the particular recipient, for example, through administration of increasing doses until a preselected desired response is achieved. It is understood that the specific dose level for any particular subject may depend upon a variety of factors including the activity of the specific compound employed, the age, body weight, general health, gender, and diet of the subject, the time of administration, the route of administration, the rate of excretion, any drug combination, and the degree of expression or activity to be modulated.

The invention further provides pharmaceutical compositions comprising two or more molecular species of the invention, each comprising a moiety that binds to a cellular marker, wherein the cell-binding moieties in each molecular species bind to a different cellular marker. The invention further provides pharmaceutical compositions comprising one or more molecular species of the invention and an additional active agent. The additional active agent may be an agent that is effective for treatment of a macular degeneration related condition, diabetic retinopathy, ONV, or ocular inflammation. In certain embodiments of the invention the additional active agent is selected from the group consisting of: angiogenesis inhibitors, antiinflammatory agents, antiangiogenic steroids, and growth factors. The additional active agent can be an antibiotic or an antiinflammatory agent not necessarily effective specifically for treatment of a macular degeneration related condition, diabetic retinopathy, or ONV.

### Examples

Example 1: Prevention of Choroidal Neovascularization in a Mouse Model by Administration of an Antagonist of a GPCR

Several GPCR antagonist compounds as discussed herein are produced as described in U.S. Pat. No. 6,821,950 and/or March, *supra,* and tested as described below. The compounds have the following sequences (i) AcF-(OPdChaWR), (ii) Suc-F-(OPdChaWR), (iii) AcF- (OPdPheWR) where abbreviations are listed above (Table 1) and ( ) denotes cyclization through an amide bond. Compounds are dissolved in sterile physiological saline. The experiment below is performed for each compound.

Mice (3 groups per dose; N=10 in each group) are anesthetized and their pupils dilated. Krypton red laser photocoagulation is used to generate multiple (e.g., 3-20) laser spots in each eye as described in Bora, 2003. For the first group, various doses of each compound (e.g., 1-100 µg) are administered by injection to one eye in each mouse at days 1 and 4 following laser treatment. The other eye serves as a control. For the second group, various doses of each compound (e.g., .01-100 mg/kg) are administered intravenously at days 1 and 14. Mice in the third group serve as a control.

In another experiment, various doses of each compound are administered at different time points following laser treatment. In other experiments, combinations of a GPCRA and VCP or SPICE are administered. The amount of VCP or SPICE can vary as for the GPCRA compound. Different concentations and amounts of each agent are tested.

Mice are sacrificed at various time points, perfused with saline containing fluorescein-labeled dextran (FITC-dextran, Sigma), their eyes enucleated, and scleralchoroidal-RPE flat mounts are prepared and stained with a mAB against elastin (Sigma) and a CY3-conjugated secondary antibody (Sigma) and examined with a confocal microscope and with light microscopy. The CNV stains green, while elastin in Bruch's membrane stains red. The number of CNV-positive laser spots and the total average area of CNV (normalizing for the number of spots) is determined. A reduced number of CNV-positive spots and/or a reduced total area of CNV in the treated eyes versus untreated eyes (both eyes of mice that were treated systemically are considered "treated") indicates that the GPCRA is effective in preventing and/or treating CNV. For example, preferably the total number of CNV-positive spots and or total average area of CNV in the treated eyes is 90% or less of the total area of CNV in the untreated eyes, preferably 70% or less, yet more prefererably 50% or less, yet more preferably 30% or less.

Flat mounts are alsostained for various drusen constituents and lipofuscin. Expression of various drusen constituents and growth factors thought to play a role in CNV is assessed in samples obtained from the eyes by RT-PCR and ELISA assay.

Example 2: Prevention of Choroidal Neovascularization in a Mouse Model by Administration of an Antagonist of a GPCRA

Several GPCR antagonist compounds as discussed herein are produced as described in U.S. Pat. No. 6,821,950 and/or March, *supra,* and/or Woodruff, *supra,* and tested as described below. One such compound referred to herein as GPCRA-1 has the sequence HCin-(OPdChaWR), where HCin = hydrocinnamate; dCHA = d-cyclohexylalanine; O =1-ornithine, and ( ) denotes cyclization through an amide bond. Compounds are dissolved in sterile physiological saline for injection or in sterile water for oral administration.

CNV induction in mice

C57BL/6 mice (The Jackson Laboratory) are anesthetized with a mixture of ketamine/xylazine (8:1) and the pupils were dilated with a single drop of 1% tropicamide. Krypton red laser photocoagulation (50-µm spot size, 0.05s duration, 250 mW) isused to generate laser spots surrounding the optic nerve by using a handheld coverslip as a contact lens. Formation of a bubble at the laser spot indicates rupture of Bruch's membrane. Multiple laser spots are generated in each eye.

Injection of GPCRA-1, or a combination of GPCRA-1 and SPICE into the eyes of mice

Mice in which CNV has been previously laser-induced are administered solutions containing GPCRA-1 or a combination of GPCRA-1 and SPICE by intravitreal injection. Different groups of mice are injected with different quantities of either or both compounds or of mouse albumin (as a control) to determine the effect of dosage on the efficacy and toxicity of the compounds. In one experiment, GPCRA-1 (30 µg) or a combination of GPCRA-1 and SPICE (15 µg each) are administered. Briefly, after anesthesia and dilation of the pupil, the anterior chamber is entered via the limbus with a 28-gauge needle to decompress the eye. Under an operating microscope, which allows visualization of the retina, a 32-gauge (blunt) needle is passed through a scleral incision, just behind the limbus, into the vitreous cavity. A Hamilton syringe is used to inject between 1 and 3 µl of a solution containing either GPCRA-1 (30 µg), SPICE (30 µg), or a combination of the two (15 µg each) or albumin.

Oral administration of GPCRA-1

Mice in which CNV has been previously laser-induced are administered solutions containing GPCRA-1 by oral gavage. Different groups of mice are treated with different quantities (ranging from 0.1 - 100 mg/kg/day) of the compound or of mouse albumin (as a control) to determine the effect of dosage on the efficacy and toxicity of the compounds.

Determination of incidence and size CNV

Seven days after CNV induction incidence of CNV is determined. Briefly, the mice are perfused with a FITC-dextran (Sigma-Aldrich) solution just prior to sacrifice. After the eyes are excised and fixed for 1 h in 10% phosphate-buffered formalin, RPE-choroid-scleral flat mounts are prepared as follows. The cornea and the lens are removed and the neurosensory retina carefully dissected from the eyecup. Five radial cuts are made from the edge of the eyecup to the equator; the sclerachoroidretinal pigment epithelium (RPE) complex is flat-mounted, with the sclera facing down, on a glass slide in Aquamount. The flat mounts are stained with an anti-elastin specific monoclonal antibody (Sigma-Aldrich) and then with a CY3-conjugated secondary antibody (Sigma-Aldrich) at a suitable concentration, e.g., at a 1/200 dilution of a 1.0 mg/ml stock solution. Mounts are observed under confocal microscopy (LSM510, Zeiss). The prominent neovascular growth stains green whereas the underlying elastin in the Bruch's membrane stains red within a laser spot. Images are analyzed with the image analysis software AxioVision (Zeiss). The amount of CNV is determined by measuring the total green-fluorescent surface area in each picture. A mean green-fluorescent area is obtained for the various groups and compared using student t-test for comparisons between groups and ANOVA for comparison among multiple groups. At least 10 spots are studied for each group. Deposition of a variety of different complement components is also measured using immunological techniques and/or RT-PCR. A reduced amount of CNV in the treated eyes versus untreated eyes (both eyes of mice that were treated orally are considered "treated") indicates that the therapy is effective in preventing and/or treating CNV. For example, preferably the total number of CNV-positive spots and or total average area of CNV in the treated eyes is 90% or less of the total area of CNV in the untreated eyes, more preferably 70% or less, yet more prefererably 50% or less.

Example 3: Treatment with a GPCR Antagonist in a Mouse Model of Age Related Macular Degeneration

GPCRAs as described in Examples 1 and 2 are individually and in combination administered to 15, 16, or 18 month old normal mice and age-matched mice deficient in Ccl-2 and/or Ccr-2 (Ambati, et al.). In addition, combinations of a GPCR antagonist and VCP or SPICE are tested using similar amounts of these agents. Administration is performed by injection to one eye. The other eye serves as a control. In another experiment the compound or compound combination is administered intravenously. Various doses (as in Example 1) and treatment regimens are used. For example, in some groups the compound(s) are injected every 3 days. In other groups the compound(s) are injected weekly. The mice are sacrificed at various time points and their eyes are processed and analysed as described in Example 1. The ability of the compounds and compound combinations to prevent and/or treat CNV, RNV, or other features of ARMD such as photoreceptor atrophy, drusen and lipofuscin accumulation, etc., is assessed as described in Examples 1 and 2.

Example 4: Treatment of ARMD with a GPCRA-1

Subjects with exudative ARMD are divided into four groups. A variety of parameters, for example visual acuity (e.g., best corrected visual acuity), contrast sensitivity, visual distortion, retinal hemorrhage number or area, macular thickness, etc., are evaluated to provide baseline indication of the condition of the subject's eye. One group receives a single intravitreal injection of a GPCRA, e.g., GPCRA-1. A second group receives SPICE. A third group receives GPCRA-1 and SPICE (each at half the dose administered to groups 1 and 2). A fourth group receives an angiogenesis inhibitor such as an anti-VEGF agent, e.g., Lucentis, Avastin, or Macugen. The compounds (100 µg) are administered by a single intravitreal injection. Identical studies are performed in parallel using 30 µg, 50 µg, and 150 µg of each compound.

The groups are monitored over time. Parameters such as visual acuity (e.g., best corrected visual acuity), contrast sensitivity, visual distortion, retinal hemorrhage number or area, macular thickness, etc., are evaluated, preferably using the same methods and metrics as were used in the initial evaluation to determine baseline values. Evaluations to determine the number of subjects who experience improvement in the condition of a treated eye can take place, e.g., 1 week, 10 days, 2, 3, 4, 5, 6, 8, 10, and/or 12 weeks following treatment. Preferably subjects are examined at regular intervals, e.g., at least every 2-4 weeks. The number of subjects that experience improvement in the condition of a treated eye is compared between the two groups. Also monitored is the average time to destabilization, e.g., the average time before an acute deterioration in one or more of the foregoing parameters occurs. Also monitored (e.g., using fluorescein angiography and/or ophthalmologic examination) is the extent of neovascularization and/or vessel leakage at various time points following treatment, e.g., at 30, 60, 90, 120, 150, and 180 day time points and at 30 day intervals thereafter (or an appropriate subset of these time points). The change from baseline in a retinal thickness score is evaluated and compared between the various groups. A greater mean decrease in retinal thickness at one or more of the foregoing time points in certain group(s) is indicative that the agent and dose administered to those group(s) combined therapy of the present invention provides a therapeutic advantage for treating the eye disorder. The change from baseline in fluorescein leakage score (where the fluorescein leakage score provides an indication of neovascularization and/or vessel leakage and a higher score indicates a greater amount of neovascularization and/or vessel leakage) may be evaluated. A greater mean decrease in fluorescein leakage score from baseline in the in in certain group(s) is indicative that the agent and dose administered to those group(s) combined therapy of the present invention provides a therapeutic advantage for treating the eye disorder. The change from baseline in a visual acuity score is evaluated and compared between the various groups. A greater mean increase in visual acuity, or a lesser decrease in visual acuity, at one or more of the foregoing time points in certain group(s) is indicative that the agent and dose administered to those group(s) may provide a therapeutic advantage for treating the eye disorder. For example, an improvement rate may be defined as the proportion of subjects who experience 2 lines or more improvement in visual acuity from baseline. The improvement rate is determined at each time point for the two groups. A higher improvement rate at one or more time points in one of the groups is indicative that the treatment administered to subjects in that group may have a therapeutic advantage for treating the eye disorder.

Example 5: Treatment of ARMD with an Injectable Ocular Implant Containing a GPCRA

Example 4 is repeated except that the compounds are administered using an intravitreal implant administered by intravitreal injection. Results achieved in the different groups are compared with each other and with results achieved in the study described in Example 4.

Example 6: Treatment of ARMD with an Ocular Implant Containing a GPCRA

Example 4 is repeated except that the compounds are administered using a surgically administered intravitreal implant, compositions comprising nanoparticles containing the therapeutic agentsResults achieved in the different groups are compared with each other and with results achieved in the studies described in Example 4 and 5.

### Equivalents and Scope

Those skilled in the art will recognize, or be able to ascertain using no more than routine experimentation, many equivalents to the specific embodiments of the invention described herein. The scope of the present invention is not intended to be limited to the above Description, but rather is as set forth in the following claims.

In the claims articles such as "a,", "an" and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process. Furthermore, it is to be understood that the invention encompasses all variations, combinations, and permutations in which one or more elements, limitations, clauses, descriptive terms, etc., from one or more of the claims is introduced into another claim unless the context or description indicates otherwise. Any claim that is dependent on another claim can be modified to include one or more elements, limitations, clauses, or descriptive terms found in any other claim that is dependent on the same base claim unless the context or description indicates otherwise. Furthermore, where the claims recite a composition, it is to be understood that methods of administering the composition according to any of the methods disclosed herein, methods of using the composition for any of the purposes disclosed herein are included, and methods of making the composition according to any of the methods of making disclosed herein are included, unless otherwise indicated or unless it would be evident to one of ordinary skill in the art that a contradiction or inconsistency would arise. Unless otherwise indicated or evident from the context, description, or understanding of one of ordinary skill in the artany composition comprising a GPCRA can comprise any GPCRA described herein in certain embodiments of the invention.

Where elements are presented as lists, e.g., in Markush group format, it is to be understood that each subgroup of the elements is also disclosed, and any element(s) can be removed from the group. It should it be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not been specifically set forth *in haec verba* herein.

Any of the embodiments of the invention that include administering a composition to a subject can include a step of providing a subject, e.g., a subject at risk of or suffering from a disease, disorder, or condition. "Providing a subject suffering from or at risk of a condition" is intended to indicate that a compound or composition is administered to a subject known or suspected to be suffering from the disease, disorder, or condition, or at increased risk of developing the disease, disorder, or condition relative to an average member of the population and is intended to indicate that the composition is administered for purposes of treating or preventing the disease, disorder, or condition and should not otherwise be construed as limiting the invention. The methods may include a step of identifying, e.g., diagnosing, a subject as suffering from or at risk of a disease, disorder, or condition.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

In addition, it is to be understood that any one or more compounds, diseases, conditions, or other claim elements may be explicitly excluded from any one or more of the claims. For purposes of brevity, these various embodiments in which one or more compounds, diseases, conditions, or other claim elements is/are excluded from the claims are not set forth individually herein but are included in the invention.

## Claims

1. A composition comprising a compound of general formula I shown below: 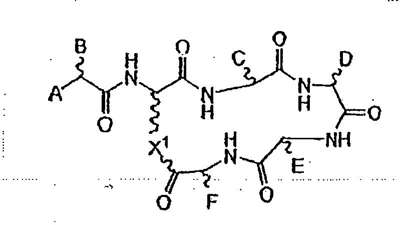 where A is H, alkyl, aryl, NH₂, NHalkyl, N(alkyl)₂, NHaryl or NHacyl; B is an alkyl, aryl, phenyl, benzyl, naphthyl or indole group, or the side chain of a D- or L-amino acid selected from the group consisting of phenylalanine, homophenylalanine, tryptophan, homotryptophan, tyrosine, and homotyrosine; C is the side chain of a D-, L- or homo- amino acid selected from the group consisting of proline, alanine, leucine, valine, isoleucine, arginine, histidine, aspartate, glutamate, glutamine, asparagine, lysine, tyrosine, phenylalanine, cyclohexylalanine, norleucine, tryptophan, cysteine and methionine; D is the side chain of a D- or L-amino acid selected from the group consisting of cyclohexylalanine, homocyclohexylalanine, leucine, norleucine, homoleucine, homonorleucine and tryptophan; E is the side chain of a D- or L-amino acid selected from the group consisting of tryptophan and homotryptophan; F is the side chain of a D- or L-amino acid selected from the group consisting of arginine, homoarginine, lysine and homolysine or is one of the following side-chains 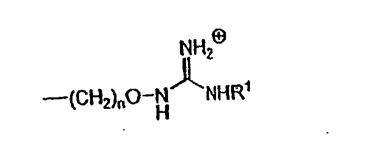 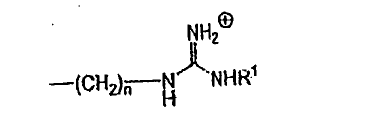 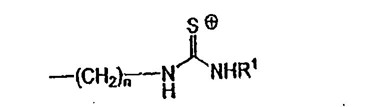 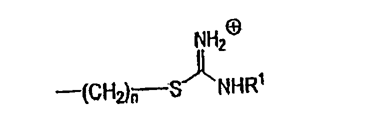 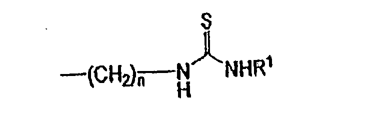 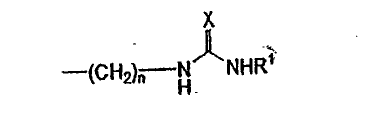 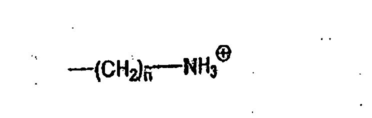 or another mimetic of an arginine side chain, where X is NCN, NNO₂, CHNO₂ or NSO₂NH₂; n is an integer from 1 to 4, and R¹ is H or an alkyl, aryl, CN, NH₂, OH, -CO-CH₂CH₃, -CO-CH₃, -CO-CH₂CH₂CH₃, -CO-CH₂ Ph, or -CO- Ph; and X¹ is ∼(CH2)ₙNH∼or (CH₂)ₙ -S-, ∼(CH₂)₂ O-, ∼(CH₂)₃ O∼, ∼(CH₂)₃ --, ∼(CH₂)₄ --, or -CH₂ COCHRNH-, where R is the side chain of any common or uncommon amino acid, and where n is an integer of from 1 to 4, e.g., 1, 2, 3, or 4 for use in treating a subject suffering from or at risk of the dry form of age-related macular degeneration, ocular neovascularization, or to treat a symptom or manifestation of the dry form of age-related macular degeneration.

2. The composition of claim 1, wherein the compound is selected from the group consisting of SEQ ID NOs: 11, 12, 13, 14, 15, 16, 17, 18, 19, 20, 21, 22, 23, 24, 25, 26, 27 and 28.

3. The composition of claim 1 , wherein n is 2 or 3.

4. The composition of claim 1, wherein F is one of the following side-chains 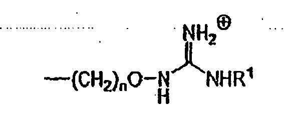 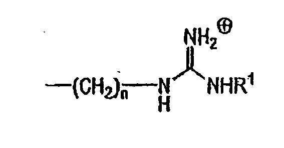 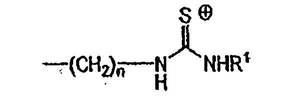 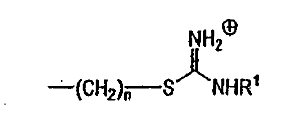 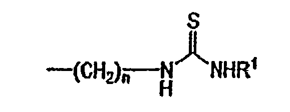 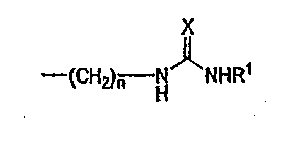 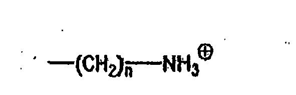 or another mimetic of an arginine side chain; where X is NCN, NNO₂ CHNO₂ or NSO₂NH₂; n is an integer from 1 to 4, and R¹ is H or an alkyl, aryl, CN, NH₂, OH, ∼CO-CH₂CH₃, -CO-CH₃, -CO-CH₂CH₂CH₃, -CO-CH₂ Ph, or -CO-Ph; B is an indole, indole methyl, benzyl, phenyl, naphthyl, naphthyl methyl, cinnamyl group, or
any other derivative of the aromatic group; and C is D- or L-cyclohexylalanine (Cha), leucine, valine, isoleucine, phenylalanine, tryptophan or methionine.

5. The composition of claim 1 , wherein the compound has the following formula:
Ac-Phe-[Lys-Pro-(dCha)-Trp-Arg] or
Ac-Phe-[Om-Pro-(dCha)-Trp-Arg] or
HCin-[Orn-Pro-(dCha)Trp-Arg] .

6. The composition of claim 1, wherein A is L-arginine.

7. The composition of claim 1, wherein F is an L-amino acid preferably wherein F is an L-arginine.

8. The composition of claim 1, wherein R¹ is methyl, ethyl, propyl, or butyl.

9. The composition of claim 1, wherein the compound is selected from SEQ ID NOs: 29, 30, 32, 33, 41, 46, 50, 52, 58, 69, and 74.

10. The composition of claim 1, wherein the composition further comprises a pharmaceutically acceptable carrier or excipient.

11. The composition of claim 1, wherein the composition is administered intravenously.

12. The composition of claim 1, wherein the composition is locally administered to the eye preferably wherein the composition is locally administered as a liquid or wherein the composition is locally administered as an ointment, gel, or eyedrops or wherein the composition is locally administered as a solid, or wherein a sustained release formulation comprising the compound is administered, or wherein an ocular implant comprising the compound is administered.

13. The composition of claim 1, wherein the subject is at risk of or suffering from dry ARMD.

14. The composition of claim 1, wherein the subject is at risk of or suffering from choroidal neovascularization, retinal neovascularization, uveitis, or diabetes.

15. The composition of claim 1, wherein the composition further comprises a moiety that binds to a component present in the eye of a subject at risk of or suffering from a macular degeneration related condition or ocular neovascularization.

16. The composition of claim 15, wherein the moiety binds to a cellular marker present on or at the surface of an endothelial cell or retinal pigment epithelial cell or wherein the moiety binds to a drusen constituent, or wherein the moiety is either directly or indirectly linked to the compound, or wherein the moiety is linked to particles that contain the compound, or wherein the moiety is or comprises sLex or a derivative thereof.

17. A composition comprising a compound having the formula 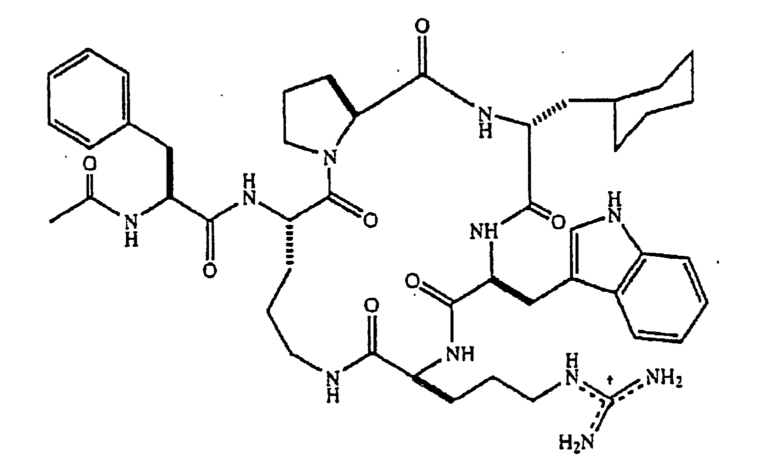 for use in treating a subject suffering from or at risk of the dry form of age-related macular degeneration, ocular neovascularization or to treat a symptom or manifestation of the dry form of age-related macular degeneration.

18. An ocular implant or polymeric delivery vehicle comprising a compound as set forth in claim 1.

19. An ocular implant of claim 19 for use in treating a macular degeneration related condition or ocular neovascularization.

20. An ocular implant or polymeric delivery vehicle comprising a compound as set forth in claim 1, 4, 5, 9 or 17.

## Patentansprüche

1. Zusammensetzung, welche eine Verbindung der folgenden allgemeinen Formel I umfasst: 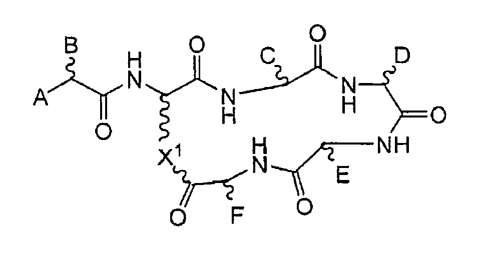 wobei A H, Alkyl, Aryl, NH₂, NH-Alkyl, N(Alkyl)₂, NH-Aryl oder NH-Acyl ist; B eine Alkyl-, Aryl-, Phenyl-, Benzyl-, Naphthyl- oder Indolgruppe oder die Seitenkette einer D- oder L-Aminosäure ist, die aus der Gruppe ausgewählt aus Phenylalanin, Homophenylalanin, Tryptophan, Homotryptophan, Tyrosin und Homotyrosin ist; C die Seitenkette einer D-, L- oder Homoaminosäure ist, die aus der Gruppe ausgewählt aus Prolin, Alanin, Leucin, Valin, Isoleucin, Arginin, Histidin, Aspartat, Glutamat, Glutamin, Asparagin, Lysin, Tyrosin, Phenylalanin, Cyclohexylalanin, Norleucin, Tryptophan, Cystein und Methionin ist; D die Seitenkette einer D- oder L-Aminosäure ist, die aus der Gruppe ausgewählt aus Cyclohexylalanin, Homocyclohexylalanin, Leucin, Norleucin, Homoleucin, Homonorleucin und Tryptophan ist; E die Seitenkette einer D- oder L-Aminosäure ist, die aus der Gruppe ausgewählt aus Tryptophan und Homotryptophan ist; F die Seitenkette einer D- oder L-Aminosäure ausgewählt aus der Gruppe bestehend aus aus Arginin, Homoarginin, Lysin und Homolysin besteht, oder eine der Seitenketten 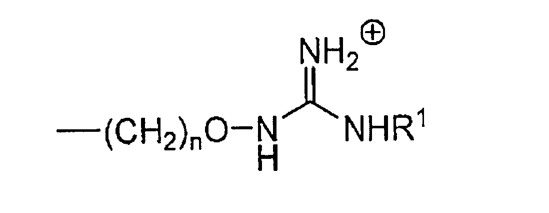 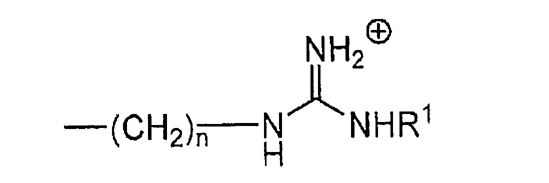 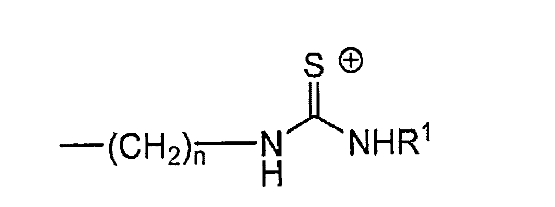 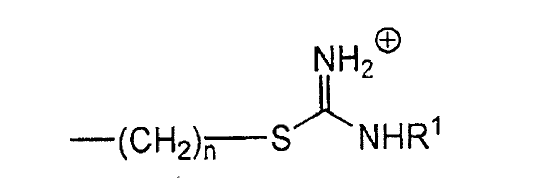 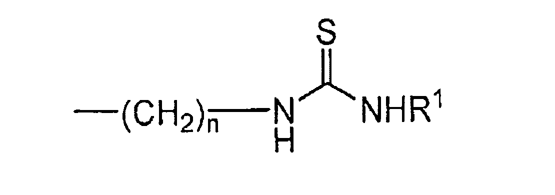 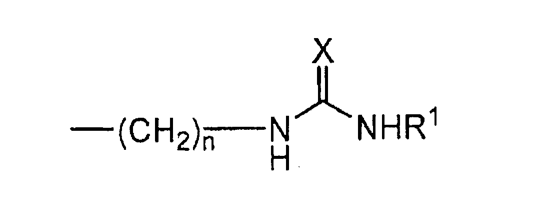 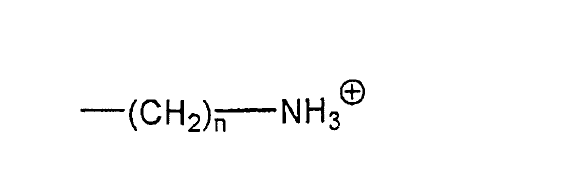 oder eine andere mimetische Gruppe für eine Arginin-Seitenkette ist, wobei X NCN, NNO₂, CHNO₂ oder NSO₂NH₂ ist; n eine ganze Zahl von 1 bis 4 ist und R¹ H oder eine Alkyl-, Aryl-, CN-, NH₂-, OH-, -CO-CH₂CH₃-, CO-CH₃-, -CO-CH₂CH₂CH₃-, CO-CH₂Ph- oder CO-Ph-Gruppe ist; und X¹ ∼(CH₂)ₙNH∼ oder (CH₂)ₙ-S-, ∼(CH₂) ₂O-, ∼(CH₂)₃O∼, ∼(CH₂)₃-, ∼(CH₂)₄- oder -CH₂COCHRNH- ist, wobei R die Seitenkette irgendeiner gewöhnlichen oder ungewöhnlichen Aminosäure ist, und wobei n eine ganze Zahl von 1 bis 4 ist, z.B. 1, 2, 3 oder 4; zur Verwendung bei der Behandlung eines Subjekts, welches an der trockenen Form einer altersbedingten Makuladegeneration oder einer okularen Neovaskularisation leidet oder ein Risiko dafür aufweist, oder zur Behandlung eines Symptoms oder einer Ausprägung der trockenen Form einer altersbedingten Makuladegeneration.

2. Zusammensetzung nach Anspruch 1, wobei die die Verbindung aus der Gruppe ausgewählt ist, die aus den SEQ-ID-Nrn. 11, 12, 13, 14, 15, 16, 17, 18, 19, 20, 21, 22, 23, 24, 25, 26, 27 und 28 besteht.

3. Zusammensetzung nach Anspruch 1, wobei n 2 oder 3 ist.

4. Zusammensetzung nach Anspruch 1, wobei F eine der folgenden Seitenketten 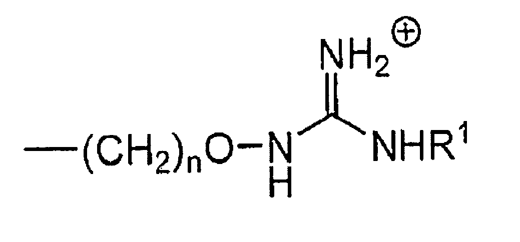 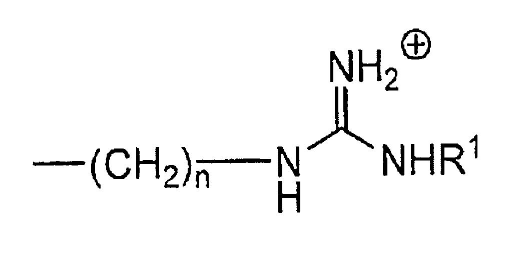 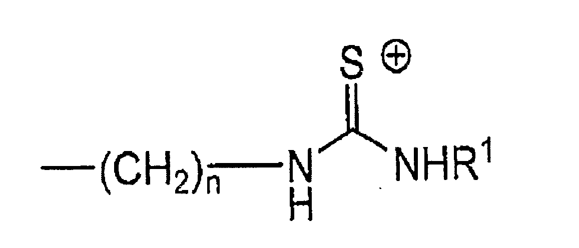 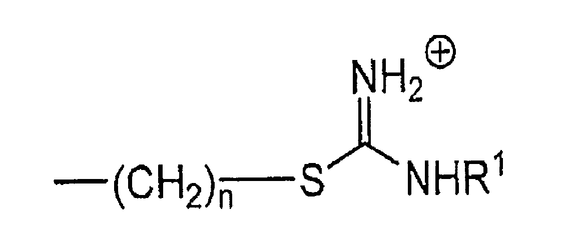 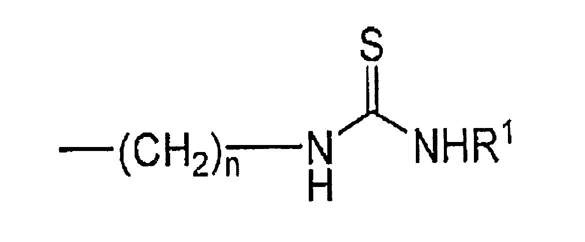 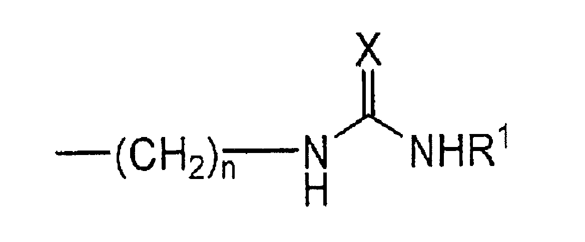 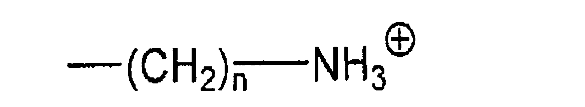 oder eine andere mimetische Gruppe für eine Arginin-Seitenkette ist, wobei X NCN, NNO₂, CHNO₂ oder NSO₂NH₂ ist, n eine ganze Zahl von 1 bis 4 ist und R¹ H oder eine Alkyl-, Aryl-, CN-, NH₂-, OH-, -CO-CH₂CH₃-, CO-CH₃-, -CO-CH₂CH₂CH₃-, CO-CH₂Ph- oder CO-Ph-Gruppe ist; B eine Indol-, Indolmethyl-, Benzyl-, Phenyl-, Naphthyl-, Naphthylmethyl-, Cinnamylgruppe oder irgendein anderes Derivat der aromatischen Gruppe ist und C D- oder L-Cyclohexylalanin (Cha), Leucin, Valin, Isoleucin, Phenylalanin, Tryptophan oder Methionin ist.

5. Zusammensetzung nach Anspruch 1, wobei die Verbindung die folgende Formel aufweist:
Ac-Phe-[Lys-Pro-(dCha)-Trp-Arg] oder
Ac-Phe-[Orn-Pro-(dCha)-Trp-Arg] oder
HCin-[Orn-Pro-(dCha)-Trp-Arg].

6. Zusammensetzung nach Anspruch 1, wobei A L-Arginin ist.

7. Zusammensetzung nach Anspruch 1, wobei F eine L-Aminosäure, vorzugsweise L-Arginin, ist.

8. Zusammensetzung nach Anspruch 1, wobei R¹ eine Methyl-, Ethyl-, Propyl- oder Butylgruppe ist.

9. Zusammensetzung nach Anspruch 1, wobei die Verbindung aus den SEQ-ID-Nrn. 29, 30, 32, 33, 41, 46, 50, 52, 58, 69 und 74 ausgewählt ist.

10. Zusammensetzung nach Anspruch 1, wobei die Zusammensetzung ferner eine pharmazeutisch unbedenkliche Trägersubstanz oder Salbengrundlage enthält.

11. Zusammensetzung nach Anspruch 1, wobei die Zusammensetzung intravenös verabreicht wird.

12. Zusammensetzung nach Anspruch 1, wobei die Zusammensetzung lokal am Auge verabreicht wird, wobei die Zusammensetzung vorzugsweise als Flüssigkeit lokal verabreicht wird, oder wobei die Zusammensetzung als Salbe, Gel oder Augentropfen lokal verabreicht wird, oder wobei die Zusammensetzung als Feststoff lokal verabreicht wird, oder wobei eine Formulierung mit verzögerter Freisetzung verabreicht wird, welche die Verbindung enthält, oder wobei ein Augenimplantat verabreicht wird, welches die Verbindung enthält.

13. Zusammensetzung nach Anspruch 1, wobei das Subjekt ein Risiko für eine trockene ARMD aufweist oder an einer solchen leidet.

14. Zusammensetzung nach Anspruch 1, wobei das Subjekt ein Risiko für eine chorioide Neovaskularisation, eine retinale Neovaskularisation, eine Uveitis oder Diabetes aufweist oder an einer solchen leidet.

15. Zusammensetzung nach Anspruch 1, wobei die Zusammensetzung ferner eine Einheit enthält, die an eine Komponente bindet, die im Auge eines Subjekts vorliegt, welches ein Risiko für einen Zustand, der mit einer Makuladegeneration in Verbindung steht, oder eine okulare Neovaskularisation aufweist oder an einem/-er solchen leidet.

16. Zusammensetzung nach Anspruch 15, wobei die Einheit an einen Zellmarker bindet, der auf oder an der Oberfläche einer Endothelzelle oder einer Netzhautpigment-Epithelzelle vorliegt, oder wobei die Einheit an einen Drusenbestandteil bindet, oder wobei die Einheit entweder direkt oder indirekt mit der Verbindung verbunden ist, oder wobei die Einheit mit Teilchen verbunden ist, welche die Verbindung enthalten, oder wobei es sich bei der Einheit um sLex oder ein Derivat davon handelt oder die Einheit sLex oder ein Derivat davon enthält.

17. Zusammensetzung, welche eine Verbindung der Formel 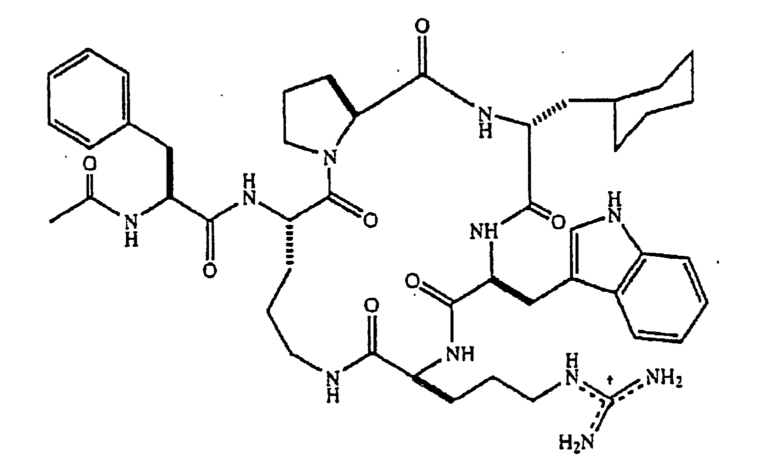 zur Verwendung bei der Behandlung eines Subjekts, welches an der trockenen Form einer altersbedingten Makuladegeneration oder einer okularen Neovaskularisation leidet oder ein Risiko dafür aufweist, oder zur Behandlung eines Symptoms oder einer Ausprägung der trockenen Form einer altersbedingten Makuladegeneration enthält.

18. Augenimplantat oder polymeres Abgabevehikel, welches eine Verbindung nach Anspruch 1 enthält.

19. Augenimplantat nach Anspruch 18 zur Verwendung bei der Behandlung eines Zustands, der mit einer Makuladegeneration in Verbindung steht, oder einer okularen Neovaskularisation.

20. Augenimplantat oder polymeres Abgabevehikel, welches eine Verbindung nach Anspruch 1, 4, 5, 9 oder 17 enthält.

## Revendications

1. Composition comprenant un composé de formule I générale présentée ci-dessous : 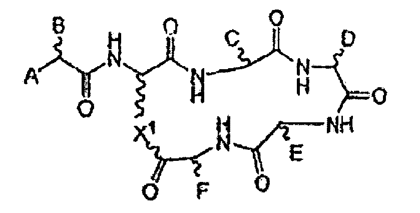 où A est H, un alkyle, un aryle, NH₂, un NHalkyle, un NH(alkyle)₂, un NHaryle ou un NHacyle ; B est un groupe alkyle, aryle, phényle, benzyle, naphtyle ou indole, ou la chaîne latérale d'un acide aminé D ou L choisi dans le groupe constitué par la phénylalanine, l'homophénylalanine, le tryptophane, l'homotryptophane, la tyrosine et l'homotyrosine ; C est la chaîne latérale d'un acide aminé D, L ou homo choisi dans le groupe constitué par la proline, l'alanine, la leucine, la valine, l'isoleucine, l'arginine, l'histidine, l'aspartate, le glutamate, la glutamine, l'asparagine, la lysine, la tyrosine, la phénylalanine, la cyclohexylalanine, la norleucine, le tryptophane, la cystéine et la méthionine ; D est la chaîne latérale d'un acide aminé D ou L choisi dans le groupe constitué par la cyclohexylalanine, l'homocyclohexylalanine, la leucine, la norleucine, l'homoleucine, l'homonorleucine et le tryptophane ; E est la chaîne latérale d'un acide aminé D ou L choisi dans le groupe constitué par le tryptophane et l'homotryptophane ; F est la chaîne latérale d'un acide aminé D ou L choisi dans le groupe constitué par l'arginine, l'homoarginine, le lysine et l'homolysine ou est l'une des chaînes latérales suivantes : 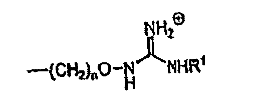 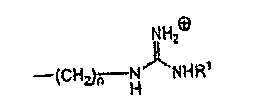 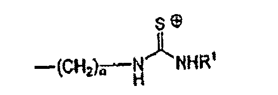 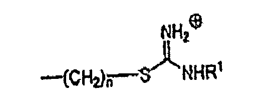 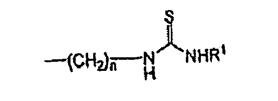 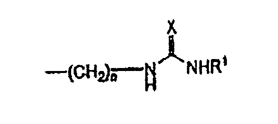 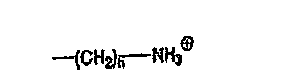 ou un autre mimétique d'une chaîne latérale d'arginine, où X est NCN, NNO₂, CHNO₂ ou NSO₂NH₂ ; n est un nombre entier allant de 1 à 4, et R¹ est H ou un alkyle, un aryle, CN, NH₂, OH, -CO-CH₂CH₃, -CO-CH₃, -CO-CH₂CH₂CH₃, -CO-CH₂Ph ou -CO-Ph ; et X¹ est ∼(CH₂)ₙNH∼ ou (CH₂)ₙ-S-, ∼(CH₂)₂O-, ∼(CH₂)₃O∼, ∼(CH₂)₃-, ∼(CH₂)₄- ou -(CH₂)COCHRNH-, où R est la chaîne latérale d'un acide aminé commun ou non commun, et où n est un nombre entier allant de 1 à 4, par exemple 1, 2, 3 ou 4, pour un usage dans le traitement d'un sujet souffrant ou risquant de souffrir de la forme sèche de la dégénérescence maculaire liée à l'âge, d'une néovascularisation oculaire, ou pour traiter un symptôme ou une manifestation de la forme sèche de la dégénérescence maculaire liée à l'âge.

2. Composition selon la revendication 1, dans laquelle le composé est choisi dans le groupe constitué par les séquences de SEQ ID NO : 11, 12, 13, 14, 15, 16, 17, 18, 19, 20, 21, 22, 23, 24, 25, 26, 27 et 28.

3. Composition selon la revendication 1, dans laquelle n est 2 ou 3.

4. Composition selon la revendication 1, dans laquelle F est l'une des chaînes latérales suivantes : 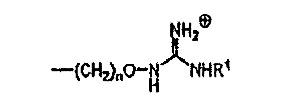 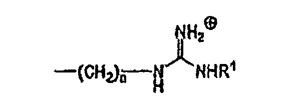 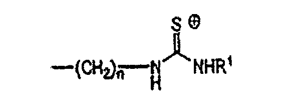 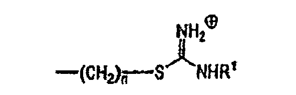 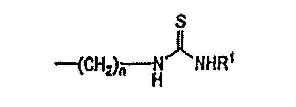 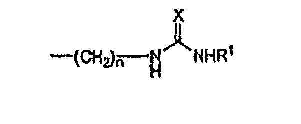 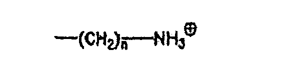 ou un autre mimétique d'une chaîne latérale d'arginine ; où X est NCN, NNO₂, CHNO₂ ou NSO₂NH₂ ; n est un nombre entier allant de 1 à 4, et R¹ est H ou un alkyle, un aryle, CN, NH₂, OH, ∼CO-CH₂CH₃, -CO-CH₃, -CO-CH₂CH₂CH₃, -CO-CH₂Ph ou -CO-Ph ; B est un groupe indole, indole méthyle, benzyle, phényle, naphtyle, naphtyl méthyle, cinnamyle, ou
tout autre dérivé du groupe aromatique ; et C est la cyclohexylalanine (Cha), la leucine, la valine, l'isoleucine, la phénylalanine, le tryptophane ou la méthionine, D ou L.

5. Composition selon la revendication 1, dans laquelle le composé possède la formule suivante :
Ac-Phe[Lys-Pro(dCha)-Trp-Arg] ou
Ac-Phe[Orn-Pro(dCha)-Trp-Arg] ou
HCin[Orn-Pro(dCha)-Trp-Arg].

6. Composition selon la revendication 1, dans laquelle A est la L-arginine.

7. Composition selon la revendication 1, dans laquelle F est un acide aminé L, de préférence dans laquelle L est la L-arginine.

8. Composition selon la revendication 1, dans laquelle R¹ est un méthyle, un éthyle, un propyle ou un butyle.

9. Composition selon la revendication 1, dans laquelle le composé est choisi parmi les séquences de SEQ ID NO : 29, 30, 32, 33, 41, 46, 50, 52, 58, 69 et 74.

10. Composition selon la revendication 1, dans laquelle la composition comprend en outre un support ou excipient pharmaceutiquement acceptable.

11. Composition selon la revendication 1, dans laquelle la composition est administrée par voie intraveineuse.

12. Composition selon la revendication 1, dans laquelle la composition est administrée localement sur l'oeil, de préférence dans laquelle la composition est administrée localement sous la forme d'un liquide ou dans laquelle la composition est administrée localement sous la forme d'une pommade, d'un gel ou de gouttes oculaires, ou dans laquelle la composition est administrée localement sous la forme d'un solide, ou dans laquelle une de formule à libération prolongée comprenant le composé est administrée, ou dans laquelle un implant oculaire comprenant le composé est administré.

13. Composition selon la revendication 1, dans laquelle le sujet souffre ou risque de souffrir d'une DMLA sèche.

14. Composition selon la revendication 1, dans laquelle le sujet souffre ou risque de souffrir d'une néovascularisation choroïdienne, d'une néovascularisation rétinienne, d'une uvéite ou de diabète.

15. Composition selon la revendication 1, dans laquelle la composition comprend en outre un fragment qui se lie à un composant présent dans l'oeil d'un sujet souffrant ou risquant de souffrir d'une affection apparentée à une dégénérescence maculaire ou d'une néovascularisation oculaire.

16. Composition selon la revendication 15, dans laquelle le fragment se lie à un marqueur cellulaire présent sur ou à la surface d'une cellule endothéliale ou d'une cellule épithéliale de pigment rétinien, ou dans laquelle le fragment se lie à un constituant de la drusen, ou dans laquelle le fragment est lié directement ou indirectement au composé, ou dans laquelle le fragment est lié à des particules contenant le composé, ou dans laquelle le fragment est ou comprend le sLex ou l'un de ses dérivés.

17. Composition comprenant un composé de formule 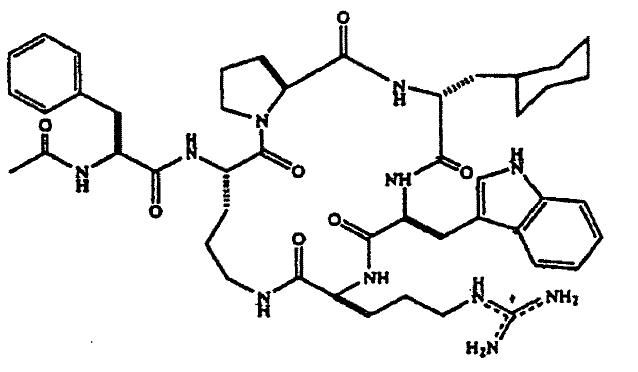 pour un usage dans le traitement d'un sujet souffrant ou risquant de souffrir de la forme sèche de la dégénérescence maculaire liée à l'âge, d'une néovascularisation oculaire, ou pour traiter un symptôme ou une manifestation de la forme sèche de la dégénérescence maculaire liée à l'âge.

18. Implant oculaire ou véhicule polymère d'administration comprenant un composé présenté dans la revendication 1.

19. Implant oculaire selon la revendication 19, utilisable pour traiter une affection apparentée à une dégénérescence maculaire ou une néovascularisation.

20. Implant oculaire ou véhicule polymère d'administration comprenant un composé présenté dans la revendication 1, 4, 5, 9 ou 17.
